# EUROPÄISCHE PATENTSCHRIFT

(11) **EP 3 634 936 B1**
(45) Veröffentlichungstag und Bekanntmachung des Hinweises auf die Patenterteilung: **06.07.2022**
(21) Anmeldenummer: 18729356.8
(22) Anmeldetag: 24.05.2018
(51) Int. Cl.: C07C 209/16, C07C 211/10, C07C 213/02, C07C 215/08, B01J 23/00

(54) **VERFAHREN ZUR HERSTELLUNG VON ETHYLENAMINEN**
PROCESS FOR PREPARING ETHYLENEAMINES
PROCÉDÉ DE LA PRÉPARATION D'ÉTHYLÈNAMINES

(30) Priorität: 09.06.2017 EP 17175144
(43) Veröffentlichungstag der Anmeldung: 15.04.2020
(73) Patentinhaber: BASF SE, 67056 Ludwigshafen am Rhein (DE)
(72) Erfinder: BEBENSEE, Regine Helga, 67056 Ludwigshafen (DE); HEIDEMANN, Thomas, 67056 Ludwigshafen (DE); BECKER, Barbara, 67056 Ludwigshafen (DE); KOCH, Eva, 67056 Ludwigshafen (DE); LUYKEN, Hermann, 67056 Ludwigshafen (DE); MELDER, Johann-Peter, 67056 Ludwigshafen (DE)
(74) Vertreter: BASF IP Association
(86) Internationale Anmeldenummer: PCT/EP2018/063613
(87) Internationale Veröffentlichungsnummer: WO 2018/224321

(56) Entgegenhaltungen:
- EP-A2- 0 839 575
- WO-A1-2013/072289

## Beschreibung

Die vorliegende Erfindung betrifft ein Verfahren zur Herstellung von Alkanolaminen und Ethylenaminen, insbesondere Ethylendiamin.

Zur großtechnischen Herstellung von Ethylendiamin (EDA) kommen in der Regel zwei Verfahren zur Anwendung.

Zum einen kann EDA durch Umsetzung 1,2 Dichlorethan mit Ammoniak unter Abspaltung von HCl hergestellt werden (EDC-Verfahren). Ein weiteres großtechnisches Verfahren zur Herstellung von EDA ist die Umsetzung von Monoethanolamin (MEA) mit Ammoniak in Gegenwart von Aminierungskatalysatoren (MEA-Verfahren).

Alternativ zu den etablierten Verfahren kann die Herstellung von EDA auch durch Umsetzung von Monoethylenglykol (MEG) mit Ammoniak erfolgen.

Ein solches Verfahren hätte verschiedene Vorteile. Ein Vorteil besteht in der guten Verfügbarkeit von MEG im Vergleich zu MEA.

MEA wird großtechnisch durch Umsetzung von Ethylenoxid (EO) und Ammoniak hergestellt. Es entsteht in der Regel ein Reaktionsgemisch, welches neben MEA auch noch höhere Ethanolamine, wie Diethanolamin (DEOA) und Triethanolamin (TEOA) enthält. Diese Nebenprodukte müssen von MEA durch einen separaten Destillationsschritt abgetrennt werden. Ethylenoxid ist ein hochentzündliches Gas, welches mit Luft explosionsfähige Gemische bilden kann. Die Handhabung von EO ist entsprechend aufwendig. Die Herstellung von MEA erfordert somit eine technisch aufwendige EO-Anlage mit anschließender Reindestillation.

Demgegenüber kann MEG sowohl auf Basis von petrochemischen Rohstoffen als auch auf Basis von nachwachsenden Rohstoffen produziert werden. Petrochemisch wird MEG ebenfalls aus EO durch Umsetzung mit Wasser hergestellt. Ebenso wie bei der Umsetzung von EO mit Ammoniak, lässt sich bei der Umsetzung von EO mit Wasser nicht verhindern, dass bereits entstandenes MEG mit EO zu Nebenprodukten, wie Di- und Triethylenglykol, reagieren kann. Die Selektivitat von MEG liegt bei ca. 90% und ist somit aber deutlich höher als die Selektivität von MEA, welche in der Regel bei 70-80% liegt. Durch den Omega-Prozess der Schell konnte die Selektivität für MEG jedoch nochmals deutlich - auf ca. 99% -- gesteigert werden. Im Omega-Proess wird EO mit CO₂ zu Ethylencarbonat umgesetzt, welches im zweiten Schritt selektiv zu MEG hydrolysiert wird.

MEG lässt sich auch über den Synthesegasweg, z.B. durch oxidative Carbonylierung von Methanol zu Dimethyloxalat und dessen anschließender Hydrierung, herstellen. Damit kommen als weiterer petrochemischer Rohstoff auch Erdgas oder Kohle für die Herstellung von MEG in Frage.

Alternativ, kann MEG auch aus nachwachsenden Rohstoffen, wie Mais oder Zuckerrohr durch Fermentation zu Ethanol, anschließender Dehydratisierung zu Ethen und nachfolgender Umsetzung mit Sauerstoff zu Ethylenoxid hergestellt werden.

Aufgrund der vielen Herstellungsvarianten ist die Verfügbarkeit von MEG im Allgemeinen hoch, was sich in der Regel positiv auf die Rohstoffkosten auswirkt.

Im Stand der Technik wird offenbart, dass die Umsetzung von MEG mit Ammoniak zu EDA sowohl in der Flüssigphase als auch in der Gasphase erfolgen kann.

Die Aminierung von MEG in der Gasphase wird in den beiden chinesischen Anmeldungen CN 102 190 588 und CN 102 233 272 offenbart.

So beschreibt die CN 102 190 588 die einstufige Umsetzung von MEG und Ammoniak in Gegenwart von Cu-haltigen Katalysatoren. Der Reaktionsdruck liegt gemäß der Beschreibung in einem Bereich von 3 bis 30 bar. Die Reaktionstemperatur liegt im Bereich von 150 bis 350°C.

In der Anmeldung CN 102 233 272 wird die Umsetzung von MEG mit Ammoniak in der Gasphase an Katalysatoren offenbart, die Cu und Ni als Hauptbestandteile und Zr, Zn, Al, Ti, Mn und Ce als Nebenkomponente beinhalten. Die Zusammensetzung der erhaltenen Reaktionsgemische wurde allerdings nicht offenbart.

Alternativ zur Umsetzung in der Gasphase kann die Umsetzung von MEG mit Ammoniak und Wasserstoff auch in der Flüssigphase erfolgen. Das Reaktionsverhalten von Katalysatoren in der Gas- und Flüssigphase unterscheidet sich jedoch in der Regel erheblich, so dass Rückschlüsse von dem Reaktionsverhalten von MEG in der Gasphase auf das Reaktionsverhalten von MEG in der Flüssigphase im Allgemeinen nicht zulässig sind.

Eine Übersicht über die metallkatalysierte Aminierung von MEG in der flüssigen Phase wird in der Diplomarbeit "Reaktionskinetische Untersuchungen zur metallkatalysierten Aminierung von Ethylenglykol in der flüssigen Phase" von Carsten Wolfgang Ihmels gegeben ("Reaktionskinetische Untersuchungen zur metallkatalysierten Aminierung von Ethylenglykol in der flüssigen Phase", Diplomarbeit der Carl von Ossietzky Universität Oldenburg vom 17.03.2000). Ihmels beschreibt eine Vielzahl von Folge- und Nebenreaktionen, die bei der Aminierung von MEG auftreten können, beispielsweise die Bildung von Di- und Triethanolamin, Disproportionierung, Nitrilbildung, Carbonylkondensation und Fragmentierungsreaktionen. Kondensation und Dispoportionierung können bei zweiwertigen Alkoholen letztendlich auch zur Bildung von Oligomeren, wie Diethylentriamin (DETA), Triethylentetramin (TETA) und Polymeren führen. Eine weitere wichtige Nebenreaktion ist die Cyclisierung. So kann Diethanolamin oder DETA zum Piperazin (PIP) weiterreagieren. Höhere Temperaturen fördern eine sich an die Cyclisierung anschließende Dehydrierung zu Aromaten. Somit wird bei der Umsetzung von MEG mit Ammoniak ein breites Produktspektrum erhalten, wobei einige Produkte in dem Produktspektrum kommerziell interessanter sind als andere. So ist für EDA, DETA und TETA der kommerzielle Bedarf höher als der von PIP oder Aminoethylethanolamin (AEEA). Gegenstand von vielen Untersuchungen bei der Umsetzung von MEG mit Ammoniak war es deshalb Katalysatoren und Reaktionsbedingungen zu finden, die zu einem vorteilhaften Produktspektrum führen.

Ihmels selber untersuchte die Umsetzung von MEG an Kobalt/Siliziumdioxid-Trägerkatalysatoren. Eine Aminierung zum gewünschten Zielprodukt MEA und EDA gelang nicht. Stattdessen bildeten sich hochpolymere Reaktionsprodukte. Unter milderen Bedingungen wurden bei noch unvollständigem Umsatz von MEG die Zielprodukte MEA und EDA in geringen Ausbeuten erhalten. Hauptprodukte waren oligomere Verbindungen.

US 4,111,840 offenbart die Umsetzung von MEG mit Ammoniak und Wasserstoff bei Drücken von 500 bis 5000 psig (ca. 34 bis 340 bar) an geträgerten Ni/Re-Katalysatoren. Dabei führten Silika/Alumina-Trägerkatalysatoren mit einer Oberflächen von 60 m²/g zu besseren Ergebnissen als Silika/Alumina-Trägerkatalysatoren mit einer spezifischen Oberfläche von 150 m²/g.

In der US 3,137,730 wird die Umsetzung von MEG mit Ammoniak in der Flüssigphase bei Temperaturen von 200-300°C und Drücken oberhalb von 1000 psig (ca. 69 bar) an Cu/Ni-Katalysatoren offenbart.

DE 1 172 268 offenbart die Umsetzung von Ethylenglykol an Katalysatoren, die mindestens eines der Metalle Cu, Ag, Mn, Fe, Ni und Co enthalten. In einem Beispiel wurde MEG mit Ammoniak bei 180°C und einem Druck von 300 bar in Gegenwart von Wasserstoff an einem CoKatalysator umgesetzt.

In der WO 2007/093514 wird ein zweistufiges Verfahren zur Herstellung von EDA offenbart, wobei in der ersten Verfahrensstufe die Aminierung an einem Hydroaminierungskatalysator bis zu einem MEA-Umsatz von maximal 40% durchgeführt wird und in der zweiten Verfahrensstufe ein geträgerter Ru/Co-Katalysatorformkörper mit kleiner Geometrie eingesetzt wird und die zweite Stufe bei einer um mindestens 10°C höheren Temperatur als die erste Verfahrensstufe durchgeführt wird.

Die WO 2013072289 offenbart die Umsetzung von Alkoholen mit einer stickstoffhaltigen Verbindung an Katalysatoren, die neben Al, Cu, Ni und Co das Element Sn beinhalten. Als bevorzugte Alkohole werden Ethylenglykol und Monoethanolamin genannt.

Katalysatoren für die Aminierung von Alkoholen, die Sn enthalten, werden ebenfalls in der WO 2011067200 offenbart. Sie dort beschriebenen Katalysatoren enthalten neben Sn auch noch die Elemente Co, Ni, Al und Cu.

Weitere Katalysatoren für die Aminierung von Alkoholen werden in der WO 200908051, der WO 2009080508, der WO 200006749 und der WO 20008006750 offenbart. Die Katalysatoren enthalten neben Zr und Ni auch Cu, Sn, Co und/oder Fe. Weitere Bestandteile sind Elemente wie V, Nb, S, O, La, B, W, Pb, Sb, Bi und In.

Die WO 9/38226 offenbart Katalysatoren für die Aminierung von Alkoholen, die Re, Ni, Co, B, Cu und/oder Ru enthalten. In einem Beispiel wird ein Träger aus SiO2 mit einer Lösung von NH4ReO4, Ni-Nitrat, H3BO3, Co-Nitrat und Cu-Nitrat getränkt und anschließend calciniert. In einem weiteren Tränkungsschritt wird der calcinierte und imprägnierte Träger mit Ru-Chlorid getränkt.

In der US 4,855,505 wird die Aminierung von MEG und MEA in Gegenwart von Katalysatoren, die Ni und/oder Co und Ru enthalten. Dabei wird ein Katalysatorvorläufer, der Ni- und/der Co-Oxid enthält mit einem Ru-Halogenid, beispielsweise Ru-Chorid, in Kontakt gebracht und anschließend im Wasserstoffstom reduziert.

Die EP 0839 575 offenbart Katalysatoren, die Co, Ni und deren Gemische und Ru auf einem porösen Metalloxidträger enthalten. Die Herstellung der Katalysatoren erfolgt durch Imprägnierung des Trägers mit den Metallen, Trocken und Calcinieren des imprägnierten Trägers und Reduktion des calcinierten Trägers in einem Wasserstoffstrom. Es wird weiterhin offenbart, dass die Imprägnierung des Trägers mit Metallverbindungen in beliebiger Reihenfolge erfolgen kann. In einem Beispiel wird ein Träger zuerst mit einer Lösung von Ni-, Co- und Cu-Nitraten imprägniert, anschließend calciniert und mit einer wässrigen Ru-Nitratlösung nachgetränkt.

Die Aufgabe der vorliegenden Erfindung bestand darin, einen heterogenen Katalysator für die Aminierung von MEG und/oder MEA in der Flüssigphase zu entwickeln, der eine ausreichende Aktivität und Selektivität bei der Umsetzung von MEG zu MEA und/odie ader EDA zeigt. Insbesondere sollte die Bildung von Wertprodukten, d.h. solchen Ethanolaminen oder Ethylenaminen mit einer hohen kommerziellen Bedeutung, insbesondere MEA und EDA, begünstigt werden und die Bildung von zyklischen Ethylenaminen, insbesondere PIP, und höheren Ethanolaminen, insbesondere AEEA, gering gehalten werden, da für PIP bzw. AEEA der kommerzielle Bedarf geringer ist als für EDA und MEA.

Insbesondere sollte auch die Konzentration bestimmter unerwünschter Nebenprodukte, wie NMEDA, NEEDA und Ethylamin (EA), verringert werden. NMEDA hat eine Flüchtigkeit, die sich kaum von EDA unterscheidet, so dass die beiden Komponenten nur mit hohem Trennaufwand zu separieren sind. Somit wäre es vorteilhaft, wenn bereits bei der Produktion nur geringe Mengen NMEDA gebildet würden. Die üblichen Produktspezifikationen von EDA erfordern, dass weniger als 500 ppm NMEDA in EDA vorhanden sein sollen.

Weiterhin sollten die Katalysatoren auch eine hohe Aktivität aufweisen und einen hohen MEG-Umsatz ermöglichen, um eine gute Raum-Zeit-Ausbeute zu erzielen.

Insgesamt sollte somit ein gutes Eigenschaftsspektrum in Bezug auf Gesamtselektivität, Selektivitätsquotient und die Bildung von unerwünschten Nebenprodukten erreicht werden.

Die Aufgabe der vorliegenden Erfindung wurde gelöst durch ein
Verfahren zur Herstellung von Alkanolaminen und/oder Ethylenaminen in der Flüssigphase, durch Umsetzung von Ethylenglykol und/oder Monoethanolamin mit Ammoniak in Gegenwart eines Aminierungskatalysators, welcher durch Reduktion eines Katalysatorvorläufers erhalten wird, dadurch gekennzeichnet, dass die Herstellung des Katalysatorvorläufers einen Schritt a) umfasst, in dem zunächst ein Katalysatorvorläufer hegestellt wird der ein oder mehrere katalytisch aktive Komponenten von Sn, Cu und Ni enthält und der in Schritt a) hergestellte Katalysatorvorläufer in einem Schritt b) gleichzeitig oder nacheinander mit einer löslichen Ru-Verbindung und einer löslichen Co-Verbindung in Kontakt gebracht wird, und worin der Katalysatorvorläufer, der in Schritt a) hergestellt wird, zusätzlich katalytisch aktive Komponenten von Co enthält.

Überraschenderweise wurde festgestellt, dass Aminierungskatalysatoren, welche erfindungsgemäß in zwei Schritten hergestellt werden, eine hohe Selektivität für die linearen Aminierungsprodukte MEA und EDA aufweisen, während die Selektivität für das zyklische Aminierungsprodukt PIP und das höhere Ethanolamin AEEA gering ist.

Weiterhin wurde festgestellt, dass durch erfindungsgemäßen Katalysatoren weniger unerwünschte Nebenprodukte, wie NMEDA, gebildet werden. Außerdem wurde festgestellt, dass die in das erfindungsgemäße Verfahren eingesetzten Aminierungskatalysatoren eine hohe Aktivität für die Umsetzung von MEG aufweisen und somit hohe Raum-Zeit-Ausbeuten bei der Umsetzung ermöglichen.

Nachfolgend und voranstehend werden folgende Abkürzungen verwendet:
- AEEA:: Aminoethylethanolamin
- AEP:: Aminoethylpiperazin
- DETA:: Diethylentriamin
- EA:: Ethylamin
- EDA:: Ethylendiamin
- EO:: Ethylenoxid
- HEP:: Hydroxyethylpiperazin
- NEEDA: N-Ethyleethylendiamin
- NMEDA:: N-Methylethylendiamin
- MEA:: Monoethanolamin
- MEG:: Monoethylenglykol
- PIP:: Piperazin
- TEPA:: Tetraethylenepentamin
- TETA:: Triethylenetetramin

### Aminierungskatalysatoren

Das erfindungsgemäße Verfahren zur Herstellung von Alkanolaminen und Ethylenaminen durch Umsetzung von MEG und/oder MEA mit NH₃ erfolgt in Gegenwart von Aminierungskatalysatoren.

### Katalysatorvorläufer

Die Aminierungskatalysatoren werden durch Reduktion von Katalysatorvorläufern erhalten.

Die Herstellung des Katalysatorvorläufers umfasst 2 Schritte.

In einem Schritt a) wird zunächst ein Katalysatorvorläufer hergestellt, der ein oder mehrere katalytisch aktive Komponenten von Sn, Cu und Ni enthält.

Dabei enthält der Katalysatorvorläufer, der in Schritt a) hergestellt wird, zusätzlich katalytisch aktive Komponenten von Co.

Der in Schritt a) erhaltene Katalysatorvorläufer wird in einem weiteren Schritt b) gleichzeitig oder nacheinander mit einer löslichen Ru-Verbindung und einer lösliche Co-Verbindung in Kontakt gebracht.

### Schritt a) Herstellung des Katalysatorvorläufers

### Aktive Masse

Die in den Schritt b) eingesetzten Katalysatorvorläufer enthalten eine aktive Masse.

Die aktive Masse der Katalysatorvorläufer enthält ein oder mehrere Aktivmetalle und optional ein oder mehrere Katalysatorzusatzelemente, sowie optional ein oder mehrere Trägermaterialien.

### Aktivmetalle

Die aktive Masse der im erfindungsgemäßen Verfahren eingesetzten Katalysatorvorläufer umfasst erfindungsgemäß ein oder mehrere Aktivmetalle ausgewählt aus der Gruppe bestehend aus Sn, Cu, und Ni.

Dabei enthält der Katalysatorvorläufer, der in Schritt a) hergestellt wird, zusätzlich katalytisch aktive Komponenten von Co,

### Katalysatorzusatzelemente

Die aktive Masse der in das erfindungsgemäße Verfahren eingesetzten Katalysatorvorläufer kann optional ein oder mehrere Katalysatorzusatzelemente umfassen.

Bei den Katalysatorzusatzelementen handelt es sich um Metalle oder Halbmetalle ausgewählt aus den Gruppen 1 bis 8, 9 , 10 (ausgenommen Ni), 11 (ausgenommen Cu) und 12 bis 13, 14 (ausgenommen Sn) und 15 bis 17 des Periodensystems, dem Element P und den Metallen der seltenen Erden.

Bevorzugte Katalysatorzusatzelemente sind Zr, Al, Fe, Sb, Pb, Bi, In, Ga, V, Nb, S, P, B, W, La, Ce, Y und Hf.

### Besonders bevorzugte Katalysatorzusatzelemente sind Zr, Al und Fe

### Katalytisch aktive Komponenten

Im Katalysatorvorläufer liegen die Aktivmetalle und die Katalysatorzusatzelemente im Allgemeinen in Form ihrer sauerstoffhaltigen Verbindungen vor, beispielsweise als Carbonate, Oxide, Mischoxide bzw. Hydroxide der Aktivmetalle bzw. Katalysatorzusatzelemente.

Die sauerstoffhaltigen Verbindungen der Aktivmetalle und der Katalysatorzusatzelemente werden nachfolgend als katalytische aktive Komponenten bezeichnet.

Der Begriff katalytisch aktive Komponenten soll aber nicht implizieren, dass diese Verbindungen an sich bereits katalytisch aktiv sind. Die katalytisch aktiven Komponenten weisen in der Regel erst nach erfolgter Reduktion des Katalysatorvorläufers eine katalytische Aktivität in der erfindungsgemäßen Umsetzung auf.

In der Regel werden die katalytisch aktiven Komponenten aus löslichen Verbindungen der Aktivmetalle bzw. der Katalysatorzusatzelemente oder ausgefällten Niederschläge der Aktimetalle bzw. der Katalysatorzusatzelemente durch eine Calcinierung in die katalytisch aktiven Komponenten umgewandelt, wobei die Umwandlung in der Regel durch Entwässerung und/oder Zersetzung erfolgt.

### Trägermaterialien

Die katalytisch aktive Masse kann weiterhin ein oder mehrere Trägermaterialien umfassen.

Bei den Trägermaterialien handelt es sich im Allgemeinen um Katalysatorzusatzelemente, die als Feststoff bei der Herstellung der Katalysatorvorläufer eingesetzt werden und auf die die löslichen Verbindungen der Aktivmetalle und/oder Katalysatorzusatzelemente aufgefällt werden oder die mit den löslichen Verbindungen der Aktivmetalle oder Katalysatorzusatelemente getränkt werden. In der Regel sind Trägermaterialien Feststoffe mit einer hohen Oberfläche.

Es werden bevorzugt Trägermaterialien eingesetzt, die bereits die nachfolgend beschriebene bevorzugte Form und Geometrie aufweisen (siehe Abschnitt Form und Geometrie der Trägermaterialien und Katalysatorvorläufer)

Auf das Trägermaterial können die katalytisch aktiven Komponenten aufgebracht werden, beispielsweise durch Auffällung der Aktivmetalle bzw. der Katalysatorzusatzelemente in Form ihrer schwerlöslichen Verbindungen, beispielsweise den Carbonaten, Hydrogencarbonaten oder Hydroxiden, oder durch Tränkung des Trägermaterials mit löslichen Verbindungen der Aktivmetalle oder der Katalysatorzusatzelemente.

Als Trägermaterial kann das Katalysatorzusatzelement Kohlenstoff, beispielsweise in Form von Graphit, Ruß und/oder Aktivkohle verwendet werden.

Bevorzugte Trägermaterialien sind Oxide der Katalysatorzusatzelemente Al, Ti, Zn, Zr und Si oder Mischungen davon, beispielsweise Aluminiumoxid (gamma, delta, theta, alpha, kappa, chi oder Mischungen daraus), Titandioxid (Anatase, Rutil, Brookit oder Mischungen daraus), Zinkoxid, Zirkondioxid Siliziumdioxid (wie Silica, pyrogenes Siliziumdioxid, Kieselgel oder Silikate), Alumosilicate, Mineralien, wie Hydrotalcit, Chrysotil und Sepiolit.

Besonders bevorzugte Trägermaterialien sind Aluminiumoxid oder Zirkonoxid oder Gemische davon.

Ein besonders bevorzugtes Trägermaterial ist Aluminiumoxid.

### Zusammensetzung der Katalysatorvorläufer

Die in das Verfahren eingesetzten Katalysatorvorläufer werden bevorzugt in Form von Katalysatorvorläufern eingesetzt, die nur aus katalytisch aktiver Masse und gegebenenfalls einem Verformungshilfsmittel (wie z. B. Graphit oder Stearinsäure), falls der Katalysatorvorläufer als Formkörper eingesetzt wird, bestehen.

Der Anteil der katalytisch aktiven Masse bezogen auf die Gesamtmasse des Katalysatorvorläufers beträgt üblicherweise 70 bis 100 Gew.-%, bevorzugt 80 bis 100 Gew.-%, mehr bevorzugt 90 bis 100 Gew.-%, noch mehr bevorzugt 95 Gew.-% bis 100 Gew.-% und besonders bevorzugt 97 Gew.-% bis 100 Gew.-%.

Die Zusammensetzung der Katalysatorvorläufer kann mittels bekannten Methoden der Elementaranalyse, beispielsweise der Atomabsorptionsspektrometrie (AAS), der Atomemissionsspektrometrie (AES), der Röntgenfluoreszenzanalyse (RFA) oder der ICP-OES (Inductively Coupled Plasma Optical Emission Spectrometry) gemessen werden.

Die Konzentrationsangaben (in Gew.-%) der katalytisch aktiven Komponenten erfolgt im Rahmen der vorliegenden Erfindung als entsprechendes Oxid.

Die Katalysatorzusatzelemente der Gruppe 1 (Alkalimetalle) werden als M₂O berechnet, beispielsweise Na₂O.

Die Katalysatorzusatzelemente der Gruppe 2 (Erdalkalimetalle) werden als MO berechnet, beispielsweise MgO oder CaO.

Die Katalysatorzusatzelemente der Gruppe 13 (Bor-Gruppe) werden M₂O₃ berechnet, beispielsweise B₂O₃ oder Al₂O₃.

In der Kohlenstoffgruppe (Gruppe 14) wird Si als SiO₂, Ge als GeO, Sn als SnO und Pb als PbO berechnet.

In der Stickstoffgruppe (Gruppe 15) wird P als H₃PO₄, As als As₂O₃, Sb als Sb₂O₃ und Bi als Bi₂O₃ berechnet.

Der der Gruppe der Chalkogene (Gruppe 16) wird Se als SeO₂ und Te als TeO₂ berechnet.

In der Scandiumgruppe (Gruppe 3) wird Sc als Sc₂O₃, Y als Y₂O₃ und La als La₂O₃ berechnet.

In der Titangruppe (Gruppe 4) wird Ti als TiO₂, Zr als ZrO₂ und Hf als HfO₂ berechnet.

In der Vanadiumgruppe (Gruppe 5) wird V als V₂O₅, Nb als Nb₂O₅ und Ta als Ta₂O₅ berechnet. In der Chromgruppe (Gruppe 6) wird Cr als CrO₂, Mo als MoO₃ und W als WO₂ berechnet.

In der Mangangruppe (Gruppe 7) wird Mn als MnO₂ und Re als Re₂O₇ berechnet.

In der Eisengruppe (Gruppe 8) wird Fe als Fe₂O₃, Ru als RuO₂ und Os als OsO₄ berechnet.

In der Cobaltgruppe (Gruppe 9) wird Co als CoO, Rh als RhO₂ und Ir als IrO₂ berechnet.

In der Nickelgruppe (Gruppe 10) wird Ni als NiO, Pd als PdO und Pt als PtO berechnet.

In der Kupfergruppe (Gruppe 11) wird Cu als CuO, Ag als AgO und Au als Au₂O₃ berechnet.

In der Zinkgruppe (Gruppe 12) wird Zn als ZnO, Cd als CdO und Hg als HgO berechnet.

Die Konzentrationsangaben (in Gew.-%) der Komponenten des Katalysatorvorläufers beziehen sich jeweils - falls nicht anders angegeben - auf die katalytisch aktive Masse des Katalysatorvorläufers nach dessen letzter Calcinierung und vor Inkontaktbringen des calcinierten Katalysatorvorläufers mit der löslichen Ru- und/oder Co-Verbindung.

Die Zusammensetzung der Katalysatorvorläufer ist im Allgemeinen abhängig von der nachfolgend beschriebenen Herstellungsmethode (Mischfällung oder Auffällung bzw. Tränkung).

Katalysatorvorläufer, die durch Mischfällung hergestellt werden enthalten kein Trägermaterial. Wenn die Fällung, wie nachfolgend beschrieben, in Gegenwart eines Trägermaterials erfolgt, so wird die Fällung im Rahmen der vorliegenden Erfindung als Auffällung bezeichnet.

Katalysatorvorläufer, die durch Mischfällung hergestellt werden, enthalten in der Regel 1 bis 3, besonders bevorzugt 1 bis 2 und insbesondere bevorzugt 1 Aktivmetall(e).

Unabhängig von der Anzahl der in der aktiven Masse vorhanden Aktivmetalle, liegt bei Katalysatorvorläufern, die durch Mischfällung hergestellt werden, die Masse der katalytisch aktiven Komponenten der Aktivmetalle bevorzugt im Bereich von 1 bis 95 Gew.-%, besonders bevorzugt 10 bis 90 Gew.-%, ganz besonders bevorzugt 20 bis 85 Gew.-% und insbesondere bevorzugt 50 bis 80 Gew.-%, bezogen auf die Gesamtmasse des Katalysatorvorläufers und wobei die katalytisch aktiven Komponenten als Oxid berechnet werden.

Katalysatorvorläufer, die durch Mischfällung hergestellt werden, enthalten in der Regel 1 bis 5, besonders bevorzugt 1 bis 4 und insbesondere bevorzugt 1 bis 3 verschiedene Katalysatorzusatzelemente.

Unabhängig von der Anzahl der in der aktiven Masse vorhanden Katalysatorzusatzelemente, liegt bei Katalysatorvorläufern, die durch Mischfällung hergestellt werden, die Masse der katalytisch aktiven Komponenten der Katalysatorzusatzelemente bevorzugt im Bereich von 1 bis 90 Gew.-%, besonders bevorzugt 5 bis 80 Gew.-% und ganz besonders bevorzugt 10 bis 60 Gew.-%, bezogen auf die Gesamtmasse des Katalysatorvorläufers und wobei die katalytisch aktiven Komponenten als Oxid berechnet werden.

Katalysatorvorläufer, die durch Auffällung hergestellt werden enthalten in der Regel 5 bis 95 Gew.%, bevorzugt 10 bis 80 Gew.-% und besonders bevorzugt 15 bis 60 Gew.-% Trägermaterial.

Katalysatorvorläufer, die durch Auffällung hergestellt werden, enthalten in der Regel 1 bis 3, besonders bevorzugt 1 bis 2 und insbesondere bevorzugt 1 Aktivmetall.

Unabhängig von der Anzahl der in der aktiven Masse vorhanden Aktivmetalle, liegt bei Katalysatorvorläufern, die durch Auffällung hergestellt werden, die Masse der katalytisch aktiven Komponenten der Aktivmetalle bevorzugt im Bereich von 5 bis 90 Gew.-%, besonders bevorzugt 10 bis 70 Gew.-% und ganz besonders bevorzugt 15 bis 60 Gew.-%, bezogen auf die Gesamtmasse des Katalysatorvorläufers und wobei die katalytisch aktiven Komponenten als Oxid berechnet werden.

Katalysatorvorläufer, die durch Auffällung hergestellt werden, enthalten in der Regel 1 bis 5, besonders bevorzugt 1 bis 4 und insbesondere bevorzugt 1 bis 3 verschiedene Katalysatorzusatzelemente.

Unabhängig von der Anzahl der in der aktiven Masse vorhanden Katalysatorzusatzelemente, liegt bei Katalysatorvorläufern, die durch Auffällung hergestellt werden, die Masse der katalytisch aktiven Komponenten der Katalysatorzusatzelemente bevorzugt im Bereich von 1 bis 80 Gew.-%, besonders bevorzugt 5 bis 70 Gew.-% und ganz besonders bevorzugt 10 bis 50 Gew.-%, bezogen auf die Gesamtmasse des Katalysatorvorläufers und wobei die katalytisch aktiven Komponenten als Oxid berechnet werden.

Katalysatorvorläufer, die durch Tränkung hergestellt werden enthalten in der Regel 50 bis 99 Gew.%, bevorzugt 75 bis 98 Gew.-% und besonders bevorzugt 90 bis 97 Gew.-% Trägermaterial.

Katalysatorvorläufer, die durch Tränkung hergestellt werden, enthalten in der Regel 1 bis 3, besonders bevorzugt 1 bis 2 und insbesondere bevorzugt 1 Aktivmetall.

Unabhängig von der Anzahl der in der aktiven Masse vorhanden Aktivmetalle, liegt bei Katalysatorvorläufern, die durch Tränkung hergestellt werden, die Masse der katalytisch aktiven Komponenten der Aktivmetalle bevorzugt im Bereich von 1 bis 50 Gew.-%, besonders bevorzugt 2 bis 25 Gew.-% und ganz besonders bevorzugt 3 bis 10 Gew.-%, bezogen auf die Gesamtmasse des Katalysatorvorläufers und wobei die katalytisch aktiven Komponenten als Oxid berechnet werden.

Katalysatorvorläufer, die durch Tränkung hergestellt werden, enthalten in der Regel 1 bis 5, besonders bevorzugt 1 bis 4 und insbesondere bevorzugt 1 bis 3 verschiedene Katalysatorzusatzelemente.

Unabhängig von der Anzahl der in der aktiven Masse vorhanden Katalysatorzusatzelemente, liegt bei Katalysatorvorläufern, die durch Tränkung hergestellt werden, die Masse der katalytisch aktiven Komponenten der Katalysatorzusatzelemente bevorzugt im Bereich von 1 bis 50 Gew.-%, besonders bevorzugt 2 bis 25 Gew.-% und ganz besonders bevorzugt 3 bis 10 Gew.-%, bezogen auf die Gesamtmasse des Katalysatorvorläufers und wobei die katalytisch aktiven Komponenten als Oxid berechnet werden.

Illustrative (nicht erfindungsgemäße) und Bevorzugte Katalysatorvorläuferzusammensetzungen Zusammensetzung 1

In einer illustrativen Ausführungsform werden Katalysatorvorläufer hergestellt, deren katalytisch aktive Masse katalytisch aktive Komponenten von Zr, Cu und Ni und ein oder mehrere katalytisch aktive Komponenten von Sb, Pb, Bi und In enthält. Solche Katalysatorvorläufer sind beispielsweise in der WO 2008/006749 offenbart.

In einer Variante dieser Ausführungsform wird ein Katalysatorvorläufer hergestellt, der
10 bis 75 Gew.-%, bevorzugt 25 bis 65 Gew.-%, besonders bevorzugt 30 bis 55 Gew.-% katalytisch aktive Komponenten des Zirkoniums, berechnet als ZrO₂;
1 bis 30 Gew.-%, bevorzugt 2 bis 25 Gew.-%, besonders bevorzugt 5 bis 15 Gew.-% katalytisch aktive Komponenten des Kupfers, berechnet als CuO,
10 bis 70 Gew.-% bevorzugt 20 bis 60 Gew.-%, besonders bevorzugt 30 bis 50 Gew.-% katalytisch aktive Komponenten des Nickels, berechnet als NiO,
0,1 bis 10 Gew.-%, besonders im Bereich von 0,2 bis 7 Gew.-%, weiter besonders im Bereich von 0,4 bis 5 Gew.-%, ganz besonders im Bereich von 2 bis 4,5 Gew.-%, katalytisch aktive Komponenten eines oder mehrerer Metalle, ausgewählt aus Sb, Pb, Bi und In, jeweils berechnet als Sb₂O₃, PbO, Bi₂O₃ bzw. In₂O₃.
enthält.

### Zusammensetzung 2

In einer bevorzugten,erfindungsgemäßen Ausführungsform werden Katalysatorvorläufer hergestellt, deren katalytisch aktive Masse katalytisch aktive Komponenten Zr, Cu, Ni und Co und ein oder mehrere katalytisch aktive Komponenten eines oder mehrerer Katalysatorzusatzelemente von Pb, Bi, Sn, Sb und In, enthält. Solche Katalysatorvorläufer sind beispielsweise in der WO 2008/006750 offenbart.

In einer besonders bevorzugten Variante dieser Ausführungsform wird ein Katalysatorvorläufer hergestellt, der
10 bis 75 Gew.-%, bevorzugt 25 bis 65 Gew.-%, besonders bevorzugt 30 bis 55 Gew.-% katalytisch aktive Komponenten des Zirkoniums, berechnet als ZrO₂,
1 bis 30 Gew.-%, bevorzugt 2 bis 25 Gew.-%, besonders bevorzugt 5 bis 15 Gew.-% katalytisch aktive Komponenten des Kupfers, berechnet als CuO, und
10 bis 70 Gew.-% bevorzugt 13 bis 40 Gew.-%, besonders bevorzugt 16 bis 35 Gew.-% katalytisch aktive Komponenten des Nickels, berechnet als NiO,
10 bis 50 Gew.-%, bevorzugt 13 bis 40 Gew.-%, besonders bevorzugt 16 bis 35 Gew.% katalytisch aktive Komponenten des Kobalts, berechnet als CoO, und
0, 1 bis 10 Gew.-%, besonders im Bereich von 0,2 bis 7 Gew.-%, weiter besonders im Bereich von 0,4 bis 5 Gew.-%, katalytisch aktive Komponenten eines oder mehrerer Metalle, ausgewählt aus Pb, Bi, Sn, Sb und In, jeweils berechnet als PbO, Bi₂O₃, SnO, Sb₂O₃ bzw. In₂O₃ enthält.

### Zusammensetzung 3

In einer weiteren, illustrativenAusführungsform werden Katalysatorvorläufer hergestellt, deren katalytisch aktive Masse katalytisch aktive Komponenten von Zr, Ni und Fe und im Bereich von 0,2 bis 5,5 Gew.-% von ein oder mehreren katalytisch aktive Komponenten von Sn, Pb, Bi, Mo, Sb und/oder P, jeweils berechnet als SnO, PbO, Bi₂O₃, MoO₃, Sb₂O₃ bzw.H₃PO₄, enthält. Solche Katalysatorvorläufer sind beispielsweise in der WO 2009/080506 offenbart.

In einer Variante dieser Ausführungsform wird ein Katalysatorvorläufer hergestellt, der
20 bis 70 Gew.-% katalytisch aktive Komponenten des Zirkoniums, berechnet als ZrO₂,
15 bis 60 Gew.-% katalytisch aktive Komponenten des Nickels, berechnet als NiO, und
0,5 bis 14 Gew.-%, bevorzugt 1,0 bis 10 Gew.-%, besonders bevorzugt 1,5 bis 6 Gew.% katalytisch aktive Komponenten des Eisens, berechnet als Fe₂O₃, und
0,2 bis 5,5 Gew.-%, bevorzugt 0,5 bis 4,5 Gew.-%, besonders bevorzugt 0,7 bis 3,5 Gew.-%, katalytisch aktive Komponenten des Zinns, Bleis, Bismuths, Molybdäns, Antimons und/oder Phosphors, jeweils berechnet als SnO, PbO, Bi₂O₃, MoO₃, Sb₂O₃ bzw. H₃PO₄,
enthält.

### Zusammensetzung 4

In einer weiteren bevorzugten, erfindungsgemäßen Ausführungsform werden Katalysatorvorläufer hergestellt, deren katalytisch aktive Masse katalytisch aktive Komponenten von Zr, Cu, Ni enthält und
im Bereich von 0,2 bis 40 Gew.-% katalytisch aktive Komponenten des Kobalts, berechnet als CoO,
im Bereich von 0, 1 bis 5 Gew.-% katalytisch aktive Komponenten des Eisens, berechnet als Fe₂O₃, und
im Bereich von 0, 1 bis 5 Gew.-% katalytisch aktive Komponenten des Bleis, Zinns, Bismuths und/oder Antimons, jeweils berechnet als PbO, SnO, Bi₂O₃ bzw. Sb₂O₃,
enthält.

Solche Katalysatorvorläufer sind beispielsweise in der WO2009/080508 offenbart.

In einer besonders bevorzugten Variante dieser Ausführungsform wird ein Katalysatorvorläufer hergestellt, der
20 bis 85 Gew.-%, besonders 25 bis 70 Gew.-%, weiter besonders 30 bis 60 Gew.-% katalytisch aktive Komponenten des Zirkoniums, berechnet als ZrO₂,
0,2 bis 25 Gew.-%, besonders 3 bis 20 Gew.-%, weiter besonders 5 bis 15 Gew.-%, katalytisch aktive Komponenten des Kupfers, berechnet als CuO,
0,2 bis 45 Gew.-%, besonders 10 bis 40 Gew.-%, weiter besonders 25 bis 35 Gew.-%, katalytisch aktive Komponenten des Nickels, berechnet als NiO,
0,2 bis 40 Gew.-%, bevorzugt 1 bis 25 Gew.-%, besonders bevorzugt 2 bis 10 Gew.-%, katalytisch aktive Komponenten des Kobalts, berechnet als CoO,
0, 1 bis 5 Gew.-%, bevorzugt 0,2 bis 4 Gew.-%, besonders bevorzugt 0,5 bis 3 Gew.-%, katalytisch aktive Komponenten des Eisens, berechnet als Fe₂O₃, und
0, 1 bis 5,0 Gew.-%, besonders 0,3 bis 4,5 Gew.-%, weiter besonders 0,5 bis 4 Gew.%, katalytisch aktive Komponenten des Bleis, Zinns, Bismuths und/oder Antimons, jeweils berechnet als PbO, SnO, Bi₂O₃ bzw. Sb₂O₃.
enthält.

### Zusammensetzung 5

In einer weiteren bevorzugten, erfindungsgemäßen Ausführungsform werden Katalysatorvorläufer hergestellt, deren katalytisch aktive Masse katalytisch aktive Komponenten Zr, Cu und Ni, und
im Bereich von 1,0 bis 5,0 Gew.-% katalytisch aktive Komponenten des Kobalts, berechnet als CoO, und
im Bereich von 0,2 bis 5,0 Gew.-% katalytisch aktive Komponenten des Vanadiums, Niobs, Schwefels, Phosphors, Galliums, Bors, Wolframs, Bleis und/oder Antimons, jeweils berechnet als V₂O₅, Nb₂O₅, H₂SO₄, H₃PO₄, Ga₂O₃, B₂O₃, WO₃, PbO bzw. Sb₂O₃,
enthält.

Solche Katalysatorvorläufer werden beispielsweise in der WO2009/080508 offenbart.

In einer besonders bevorzugten Variante dieser Ausführungsform wird ein Katalysatorvorläufer hergestellt, der
46 bis 65 Gew.-%, besonders 47 bis 60 Gew.-%, weiter besonders 48 bis 58 Gew.-%, katalytisch aktive Komponenten des Zirkoniums, berechnet als ZrO₂,
5,5 bis 18 Gew.-%, besonders 6 bis 16 Gew.-%, weiter besonders 7 bis 14 Gew.-%, katalytisch aktive Komponenten des Kupfers, berechnet als CuO,
20 bis 45 Gew.-%, besonders 25 bis 40 Gew.-%, weiter besonders 30 bis 39 Gew.-%, katalytisch aktive Komponenten des Nickels, berechnet als NiO,
1,0 bis 5,0 Gew.-%, besonders im Bereich von 1,5 bis 4,5 Gew.-%, weiter besonders im Bereich von 2,0 bis 4,0 Gew.-%, katalytisch aktive Komponenten des Kobalts, berechnet als CoO, und
0,2 bis 5,0 Gew.-%, besonders 0,3 bis 4,0 Gew.-%, weiter besonders 0,5 bis 3,0 Gew.%, katalytisch aktive Komponenten des Vanadiums, Niobs, Schwefels, Phosphors, Galiums, Bors, Wolframs, Bleis und/oder Antimons, jeweils berechnet als V₂O₅, Nb₂O₅, H₂SO₄, H₃PO₄, Ga₂O₃, B₂O₃, WO₃, PbO bzw. Sb₂0₃.

### Zusammensetzung 6

In einer weiteren bevorzugten, erfindungsgemäßen Ausführungsform werden Katalysatorvorläufer hergestellt, deren katalytisch aktive Masse katalytisch aktive Komponenten von Al, Cu, Ni, Co und Sn und
im Bereich von 0,2 bis 5,0 Gew.-% katalytisch aktive Komponenten des Yttriums, Lanthans, Cers und/oder Hafniums, jeweils berechnet als Y₂O₃, La₂O₃, Ce₂O₃ bzw. Hf₂O₃, enthält.

Solche Katalysatorvorläufer werden beispielsweise in der WO 2011/067200 offenbart.

In einer besonders bevorzugten Variante dieser Ausführungsform wird ein Katalysatorvorläufer hergestellt, der
0,2 bis 5,0 Gew.-%, besonders im Bereich von 0,4 bis 4,0 Gew.-%, weiter besonders im Bereich von 0,6 bis 3,0 Gew.-%, weiter besonders bevorzugt im Bereich von 0,7 bis 2,5 Gew.-%, katalytisch aktive Komponenten des Zinns, berechnet als SnO,
10 bis 30 Gew.-%, weiter besonders im Bereich von 12 bis 28 Gew.-%, ganz besonders 15 bis 25 Gew.-%, katalytisch aktive Komponenten des Kobalts, berechnet als CoO,
15 bis 80 Gew.-%, besonders 30 bis 70 Gew.-%, weiter besonders 35 bis 65 Gew.-%, katalytisch aktive Komponenten des Aluminiums, berechnet als Al₂O₃,
1 bis 20 Gew.-%, besonders 2 bis 18 Gew.-%, weiter besonders 5 bis 15 Gew.-%, katalytisch aktive Komponenten des Kupfers, berechnet als CuO, und
5 bis 35 Gew.-%, besonders 10 bis 30 Gew.-%, weiter besonders 12 bis 28 Gew.-%, ganz besonders 15 bis 25 Gew.-%, katalytisch aktive Komponenten des Nickels, berechnet als NiO, 0,2 bis 5,0 Gew.-%, besonders im Bereich von 0,4 bis 4,0 Gew.-%, weiter besonders im Bereich von 0,6 bis 3,0 Gew.-%, weiter besonders bevorzugt im Bereich von 0,7 bis 2,5 Gew.-%, katalytisch aktive Komponenten des Yttriums, Lanthans, Cers und/oder Hafniums, jeweils berechnet als Y₂O₃, La₂O₃, Ce₂O₃ bzw. Hf₂O₃.
enthält.

### Zusammensetzung 7

In einer Ausführungsform werden Katalysatorvorläufer eingesetzt, die dadurch hergestellt werden, dass eine Lösung (L) enthaltend Zinnnitrat und mindestens einen Komplexbildner auf den Träger aufgebracht werden, wobei die Lösung (L) keinen Feststoff oder einen Feststoffanteil von maximal 0,5 Gew.-% bezogen auf die Gesamtmenge an gelösten Komponenten enthält und die Lösung (L) zusätzlich mindestens ein weiteres Nickelsalz, Cobaltsalz und/oder Kupfersalz enthält, besonders bevorzugt Nickelnitrat, Cobaltnitrat und/oder Kupfernitrat enthält. Solche Katalysatorvorläufer sind beispielsweise in der WO 2013/072289 offenbart.

In einer bevorzugten, erfindungsgemäßen Variante dieser Ausführungsform wird ein Katalysatorvorläufer hergestellt, der
0,2 bis 5 Gew.-% katalytisch aktive Komponenten des Zinn, berechnet als SnO
15 bis 80 Gew.-% katalytisch aktive Komponenten des Aluminium, berechnet als Al₂O₃
1 bis 20 Gew.-% katalytisch aktive Komponenten des Kupfer, berechnet als CuO
5 bis 35 Gew.-% katalytisch aktive Komponenten des Nickel, berechnet als NiO, und
5 bis 35 Gew.-% katalytisch aktive Komponenten des Cobalt, berechnet als CoO
enthält.

In einer ganz besonders bevorzugten Variante dieser Ausführungsform werden Katalysatorvorläufer mit der zuvor genannten Zusammensetzung dadurch erhalten, dass lösliche Verbindungen von Co und Sn auf ein fein dispergiertes Trägermaterial aufgefällt werden, wobei die lösliche Verbindung Sn-Nitrat ist und die Auffällung in Gegenwart eines Komplexbildners erfolgt. Die lösliche Verbindung von Co ist vorzugsweise Co-Nitrat.

Die Auffällung erfolgt weiterhin bevorzugt in Gegenwart mindestens einer weiteren löslichen Verbindung eines Katalysatorzusatzelements, vorzugsweise einer löslichen Verbindung von Cu und/oder Ni. Weiterhin bevorzugt werden die Katalysatorzusatzelemente ebenfalls in Form ihrer Nitrate oder Nitrosylnitrate eingesetzt.

Der Komplexbildner wird bevorzugt ausgewählt aus der Gruppe bestehend aus Glykolsäure, Milchsäure, Hydracylsäure, Hydroxybuttersäure, Hydroxyvaleriansäure, Äpfelsäure, Mandelsäure, Citronensäure, Zuckersäuren, Tartronsäure, Weinsäure, Oxalsäurde,Malonsäure, Maleinsäure, Bernsteinsäure, Glutarsäure, Adipinsäure, Glycin, Hippursäure, EDTA, Alanin, Valin, Leucin oder Isoleucin.

Das Trägermaterial ist bevorzugt Aluminiumoxid oder Zirkonoxid oder ein Gemisch davon.

Der der mittlere Durchmesser d₅₀ der Partikeln des eingesetzten Trägermaterials liegt bevorzugt im Bereich von 1 bis 500 µm, bevorzugt 3 bis 400 µm und besonders bevorzugt 5 bis 300 µm liegt.

Die Standardabweichung des Teilchendurchmessers liegt in der Regel im Bereich von 5 bis 200%, bevorzugt 10 bis 100% und insbesondere bevorzugt 20 bis 80% des mittleren Durchmessers d₅₀.

Im Anschluss an die Auffällung wird der Katalysatorvorläufer in der Regel, wie nachfolgend beschrieben, aufgearbeitet, in dem Katalysatorvorläufer von der Lösung aus der die Auffällung erfolgte abgetrennt, gewaschen, getrocknet, calciniert und ggf. in einem Formgebungsschritt in die gewünschte Form gebracht wird.

Bevorzugt erfolgt nach der Calcinierung ein Formgebungsschritt, in dem der Katalysatorvorläufer zu Formkörpern, insbesondere Tabletten, verarbeitet wird.

Die Höhe der Tabletten liegt bevorzugt im Bereich von 1 bis 10 und besonders bevorzugt im Bereich von 1,5 bis 3 mm. Das Verhältnis von Höhe h der Tablette zum Durchmesser D der

Tablette beträgt bevorzugt, 1:1 bis 1:5, besonders bevorzugt 1:1 bis 2,5 und ganz besonders bevorzugt 1:1 bis 1:2.

### Herstellung der Katalysatorvorläufer

Die Katalysatorvorläufer können nach bekannten Verfahren, z.B. durch Fällungsreaktionen (z.B. Mischfällung oder Auffällung) oder Tränkung, hergestellt werden.

### Fällungsreaktionen - Mischfällung

Katalysatorvorläufer können über eine gemeinsame Fällung (Mischfällung) von löslichen Verbindungen der Aktivmetalle bzw. Katalysatorzusatzelemente mit einem Fällungsmittel hergestellt. Dazu wird in der Regel eine oder mehrere lösliche Verbindung der entsprechenden Aktivmetalle und ggf. eine oder mehrere lösliche Verbindungen der Katalysatorzusatzelemente in einer Flüssigkeit in der Wärme und unter Rühren so lange mit einem Fällungsmittel versetzt, bis die Fällung vollständig ist.

Als Flüssigkeit wird in der Regel Wasser eingesetzt.

Als lösliche Verbindung der Aktivmetalle, kommen üblicherweise die entsprechenden Metallsalze, wie die Nitrate oder Nitrosylnitrate, Chloride, ,Sulfate, Carboxylate, insbesondere der Acetate, oder Nitrate oder Nitrosylnitrate, der voranstehend genannten Metalle in Betracht.

Als lösliche Verbindungen der Katalysatorzusatzelemente werden in der Regel wasserlösliche Verbindungen der Katalysatorzusatzelemente, beispielsweise die wasserlöslichen Nitrate oder Nitrosylnitrate, Chloride, Sulfate, Carboxylate, insbesondere die Acetate oder Nitrate oder Nitrosylnitrate eingesetzt.

### Fällungsreaktionen - Auffällung

Katalysatorvorläufer können weiterhin durch Auffällung hergestellt werden.

Unter Auffällung wird eine Herstellmethode verstanden, bei der ein oder mehrere Trägermaterialien in einer Flüssigkeit suspendiert werden und nachfolgend lösliche Verbindungen der Aktivmetalle, wie lösliche Metallsalze der Aktivmetalle, und optional lösliche Verbindungen der Katalysatorzusatzelemente zugegeben werden, welche dann durch Zugabe eines Fällungsmittels auf das suspendierten Trägermaterial aufgefällt werden (z.B. beschrieben in EP-A2-1 106 600, Seite 4, und A. B. Stiles, Catalyst Manufacture, Marcel Dekker, Inc., 1983, Seite 15).

Als lösliche Verbindungen der Aktivmetalle bzw. Katalysatorzusatzelemente werden in der Regel wasserlösliche Verbindungen der Aktivmetalle bzw. Katalysatorzusatzelemente, beispielsweise die wasserlöslichen Nitrate oder Nitrosylnitrate, Chloride, Sulfate, Carboxylate, insbesondere die Acetate oder Nitrate oder Nitrosylnitrate eingesetzt.

Das Trägermaterial liegt in der Regel als Pulver oder Splitt vor.

Die Größe der Partikel liegt in der Regel im Bereich von 50 bis 2000 µm, bevorzugt 100 bis 1000 µm und besonders bevorzugt 300 bis 700 µm.

Die Trägermaterialien, die bei der Auffällung verwendet werden, können beispielsweise in Form von Splitt, Pulvern oder Formkörpern, wie Strängen, Tabletten, Kugeln oder Ringen, eingesetzt werden. Es werden bevorzugt Trägermaterialien eingesetzt, die bereits die nachfolgend beschriebene bevorzugte Form und Geometrie aufweisen (siehe Abschnitt Form und Geometrie der Trägermaterialien und Katalysatorvorläufer).

Als Flüssigkeit, in der das Trägermaterial suspendiert wird, wird üblicherweise Wasser eingesetzt.

### Fällungsreaktionen - Allgemein

Üblicherweise werden bei den Fällungsreaktionen die löslichen Verbindungen der Aktivmetalle bzw. Katalysatorzusatzelemente durch Zugabe eines Fällungsmittels als schwer- oder unlösliche, basische Salze gefällt.

Als Fällungsmittel werden bevorzugt Laugen, insbesondere Mineralbasen, wie Alkalimetallbasen eingesetzt. Beispiele für Fällungsmittel sind Natriumcarbonat, Natriumhydroxid, Kaliumcarbonat oder Kaliumhydroxid.

Als Fällungsmittel können auch Ammoniumsalze, beispielsweise Ammoniumhalogenide, Ammoniumcarbonat, Ammoniumhydroxid oder Ammoniumcarboxylate eingesetzt werden.

Die Fällungsreaktionen können z.B. bei Temperaturen von 20 bis 100 °C, besonders 30 bis 90 °C, insbesondere bei 50 bis 70 °C, durchgeführt werden.

Die bei den Fällungsreaktionen erhaltenen Niederschläge sind im Allgemeinen chemisch uneinheitlich und enthalten in der Regel Mischungen der Oxide, Oxidhydrate, Hydroxide, Carbonate und/oder Hydrogencarbonate der eingesetzten Metalle bzw. Halbmetalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überlässt.

### Tränkung

Die Katalysatorvorläufer können auch durch Tränkung von Trägermaterialien mit löslichen Verbindungen der Aktivmetalle bzw. Katalysatorzusatzelemente hergestellt werden (Imprägnierung oder Tränkung).

Die Trägermaterialien, die bei der Tränkung verwendet werden, können beispielsweise in Form von Splitt, Pulvern oder Formkörpern, wie Strängen, Tabletten, Kugeln oder Ringen, eingesetzt werden. Es werden bevorzugt Trägermaterialien eingesetzt, die bereits die nachfolgend beschriebene bevorzugte Form und Geometrie der Formkörper aufweisen (siehe Abschnitt Form und Geometrie der Trägermaterialien und Katalysatorvorläufer).

Die Tränkung der obengenannten Trägermaterialien kann nach den üblichen Verfahren erfolgen (A. B. Stiles, Catalyst Manufacture - Laboratory and Commercial Preperations, Marcel Dekker, New York, 1983), beispielsweise durch Aufbringung eines Salzes der Aktivmetalle oder Katalysatorzusatzelemente in einer oder mehreren Tränkstufen.

Als Salze der Aktivmetalle bzw. der Katalysatorzusatzelemente kommen in der Regel wasserlösliche Salze, wie die Carbonate, Nitrate oder Nitrosylnitrate, Carboxylate, insbesondere die Nitrate oder Nitrosylnitrate, Acetate oder Chloride der entsprechenden Aktivmetalle bzw. Katalysatorzusatzelemente in Betracht, die sich in der Regel unter den Bedingungen der Calcinierung zumindest teilweise in die entsprechenden Oxide oder Mischoxide umwandeln.

Die Tränkung kann auch nach der sogenannten "incipient wetness-Methode" erfolgen, bei der das Trägermaterial entsprechend seiner Wasseraufnahmekapazität maximal bis zur Sättigung mit der Tränklösung befeuchtet wird oder das Trägermaterial mit der Tränklösung besprüht wird. Die Tränkung kann aber auch in überstehender Lösung erfolgen.

Bei mehrstufigen Tränkverfahren ist es zweckmäßig, zwischen einzelnen Tränkschritten zu trocknen und ggf. zu calcinieren. Die mehrstufige Tränkung ist vorteilhaft dann anzuwenden, wenn das Trägermaterial in größerer Menge mit Salzen beaufschlagt werden soll.

Zur Aufbringung mehrerer Aktivmetalle und/oder Katalysatorzusatzelemente und/oder basischer Elemente auf das Trägermaterial, kann die Tränkung gleichzeitig mit allen Salzen oder in beliebiger Reihenfolge der einzelnen Salze nacheinander erfolgen.

### Aufarbeitung der Katalysatorvorläufer

Die nach diesen Tränkungsverfahren erhaltenen getränkten Katalysatorvorläufer oder die nach den Fällungsverfahren erhaltenen Niederschläge werden üblicherweise verarbeitet, indem sie von der Flüssigkeit, in der die Tränkung bzw. Fällung durchgeführt wurde abgetrennt werden, gewaschen, getrocknet, calciniert und ggf. konditioniert und einem Formgebungsprozess unterzogen werden.

### Abtrennung und Waschen

Die getränkten Katalysatorvorläufer oder die nach den Fällungsverfahren erhaltenen Niederschläge werden in der Regel von der Flüssigkeit, in der die Herstellung der Katalysatorvorläufer erfolgte, abgetrennt und gewaschen.

Verfahren zur Abtrennung und Waschen der Katalysatorformläufer sind beispielsweise aus dem Artikel "Heterogenous Catalysis and Solid Catalysts, 2. Development and Types of Solid Catalysts", in Ullmann's Encyclopedia of Industrial Chemistry (DOI: 10.1002/14356007.o05_o02) bekannt.

Als Waschflüssigkeit wird in der Regel eine Flüssigkeit verwendet, in der der abgetrennte Katalysatorvorläufer schlecht löslich ist, die aber ein gutes Lösungsmittel für am Katalysator anhaftende Verunreinigungen, beispielsweise Fällungsmittel, darstellt. Eine bevorzugte Waschflüssigkeit ist Wasser.

Bei der Batch-Herstellung erfolgt die Abtrennung in der Regel mit Rahmen-Filterpressen. Das Waschen des Filterrückstands mit Waschflüssigkeit kann hierbei durch Durchleiten der Waschflüssigkeit in Gegenstromrichtung zur Filtrationsrichtung erfolgen.

Bei der kontinuierlichen Herstellung erfolgt die Abtrennung in der Regel mit Drehtrommel-Vakuumfiltern. Das Waschen des Filterrückstands erfolgt üblicherweise dadurch, dass der Filterrückstand mit der Waschflüssigkeit besprüht wird.

Die Abtrennung des Katalysatorvorläufers kann auch durch Zentrifugation erfolgen. In der Regel erfolgt das Waschen hierbei durch Zugabe von Waschflüssigkeit beim zentrifugieren.

### Trocknen

Der abgetrennte Katalysatorvorläufer wird in der Regel getrocknet.

Verfahren zur Trocknung der Katalysatorvorläufer sind beispielsweise aus dem Artikel "Heterogenous Catalysis and Solid Catalysts, 2. Development and Types of Solid Catalysts", in Ullmann's Encyclopedia of Industrial Chemistry (DOI: 10.1002/14356007.o05_o02) bekannt. Die Trocknung erfolgt dabei bei Temperaturen im Bereich von bevorzugt 60 bis 200 °C, insbesondere von 80 bis 160 °C und besonders bevorzugt von 100 bis 140 °C, wobei die Trocknungsdauer bevorzugt bei 6 h oder mehr, beispielsweise im Bereich von 6 bis 24 h, liegt. Es sind jedoch in Abhängigkeit vom Feuchtigkeitsgehalt des zu trocknenden Materials auch kürzere Trockenzeiten wie beispielsweise ungefähr 1, 2, 3, 4, oder 5 h möglich.

Die Trocknung des abgetrennten und gewaschenen Katalysatorvorläufers kann beispielsweise in Kammeröfen, Trommeltrocknern, Drehrohröfen oder Bandtrocknern erfolgen.

Die Trocknung des Katalysatorvorläufers kann auch durch Sprühtrocknung einer Suspension des Katalysatorvorläufers erfolgen.

### Calcinierung

In der Regel werden die Katalysatorvorläufer im Anschluss an die Trocknung calciniert. Während der Calcinierung werden thermisch labile Verbindungen der Aktivmetalle bzw. Katalysatorzusatzelemente, wie Carbonate, Hydrogencarbonate, Nitrate oder Nitrosylnitrate, Chloride, Carboxylate, Oxidhydratr oder Hydroxide, zumindest teilweise in die entsprechenden Oxide und/oder Mischoxide umgewandelt.

Die Calcinierung erfolgt in der Regel bei einer Temperatur im Bereich von 250 bis 1200 °C, bevorzugt 300 bis 1100°C und insbesondere von 500 bis 1000 °C.

Die Calcinierung kann unter jeder geeigneten Gasatmosphäre erfolgen, wobei Luft und/oder Luftgemische, wie Magerluft, bevorzugt sind. Das Calcinieren kann aber auch in Gegenwart von Wasserstoff, Stickstoff, Helium, Argon und/oder Dampf oder Gemischen hiervon erfolgen.

Die Calcinierung erfolgt in der Regel in einem Muffelofen, einem Drehrohrofen und/oder einem Bandkalzinierofen durchgeführt, wobei die Calcinierungsdauer bevorzugt bei 1 h oder mehr, besondersbevorzugt im Bereich von 1 bis 24 h und ganz besonders bevorzugt im Bereich von 2 bis 12 h liegt.

### Form und Geometrie der Trägermaterialien bzw. Katalysatorvorläufer

Die Katalysatorvorläufer bzw. das Trägermaterial werden bevorzugt in Form von Pulver oder Splitt oder in Form von Formkörpern eingesetzt werden.

Wird der Katalysatorvorläufer als Pulver bzw. Splitt eingesetzt, so liegt der mittlere Durchmesser der Partikel d₅₀ in der Regel im Bereich von 50 bis 2000 µm, bevorzugt 100 bis 1000 µm und besonders bevorzugt 300 bis 700 µm. Die Standardabweichung des Teilchendurchmessers liegt in der Regel im Bereich von 5 bis 200%, bevorzugt 10 bis 100% und insbesondere bevorzugt 20 bis 80% des mittleren Durchmessers d₅₀

In einer besonders bevorzugten Ausführungsform liegt der mittlere Durchmesser d₅₀ der Partikeln des eingesetzten Pulvers oder Splitts im Bereich von 1 bis 500 µm, bevorzugt 3 bis 400 µm und besonders bevorzugt 5 bis 300 µm. Die Standardabweichung des Teilchendurchmessers liegt in der Regel im Bereich von 5 bis 200%, bevorzugt 10 bis 100% und insbesondere bevorzugt 20 bis 80% des mittleren Durchmessers d₅₀.

Wird der Katalysatorvorläufer als Formköper eingesetzt, so wird dieser bevorzugt in Form von Tabletten eingesetzt.

Die Höhe der Tabletten liegt bevorzugt im Bereich von 1 bis 10 und besonders bevorzugt im Bereich von 1,5 bis 3 mm. Das Verhältnis von Höhe h der Tablette zum Durchmesser D der

Tablette beträgt bevorzugt, 1:1 bis 1:5, besonders bevorzugt 1:1 bis 2,5 und ganz besonders bevorzugt 1:1 bis 1:2.

Die Trägermaterialien bzw. Katalysatorvorläufer können jedoch auch bevorzugt in Form von Formkörpern in das erfindungsgemäße Verfahren eingesetzt.

Als Formkörper eignen sich Formkörper mit beliebiger Geometrie bzw. Form. Bevorzugte Formen sind Tabletten, Ringe, Zylinder, Sternstränge, Wagenräder oder Kugeln, besonders bevorzugt sind Tabletten, Ringe, Zylinder, Kugeln oder Sternstränge. Ganz besonders bevorzugt ist die Zylinderform

Bei Kugeln beträgt der Durchmesser der Kugelform bevorzugt 20 mm oder weniger, besonders bevorzugt 10 mm oder weniger, ganz besonders bevorzugt 5 mm oder weniger und insbesondere bevorzugt 3 mm oder weniger.

In einer bevorzugten Ausführungsform liegt bei Kugeln der Durchmesser der Kugelform bevorzugt im Bereich von 0,1 bis 20, besonders bevorzugt 0,5 bis 10 mm, ganz besonders bevorzugt 1 bis 5 mm und insbesondere besonders bevorzugt 1,5 bis 3 mm.

Bei Strängen oder Zylindern liegt das Verhältnis von Länge : Durchmessers bevorzugt im Bereich von 1:1 bis 20:1, besonders bevorzugt 1:1 bis 14:1, ganz besonders bevorzugt im Bereich von 1:1 bis 10:1 und insbesondere bevorzugt im Bereich von 1:2 bis 6:1.

Der Durchmesser der Stränge oder Zylinder beträgt bevorzugt 20 mm oder weniger, besonders bevorzugt 15 mm oder weniger, ganz besonders bevorzugt 10 mm oder weniger und insbesondere bevorzugt 3 mm oder weniger.

In einer bevorzugten Ausführungsform liegt der Durchmesser der Stränge oder Zylinder vorzugsweise im Bereich von 0,5 bis 20 mm, besonders bevorzugt im Bereich von 1 bis 15 mm, ganz besonders bevorzugt im Bereich von 1,5 bis 10 mm.

Bei Tabletten beträgt die Höhe h der Tablette vorzugsweise 20 mm oder weniger, besonders bevorzugt 10 mm oder weniger, ganz besonders bevorzugt 5 mm oder weniger und insbesondere bevorzugt 3 mm oder weniger.

In einer bevorzugten Ausführungsform liegt die Höhe h der Tablette bevorzugt im Bereich von 0,1 bis 20 mm, besonders bevorzugt im Bereich von 0,5 bis 15 mm, ganz besonders bevorzugt im Bereich von 1 bis 10 mm und insbesondere bevorzugt im Bereich von 1,5 bis 3 mm.

Das Verhältnis von Höhe h (bzw. Dicke) der Tablette zum Durchmesser D der Tablette beträgt bevorzugt 1:1 bis 1:5, besonders bevorzugt 1:1 bis 1:2,5, ganz besonders bevorzugt 1:1 bis 1:2. Der eingesetzte Formkörper weist bevorzugt eine Schüttdichte (nach EN ISO 6) im Bereich von 0,1 bis 3 kg/l, bevorzugt von 1,0 bis 2,5 kg/l und insbesondere bevorzugt 1,2 bis 1,8 kg/l auf.

### Formgebung

Bei der Herstellung der Katalsatorvorläufer durch Tränkung bzw. durch Auffällung werden bevorzugt Trägermaterialien eingesetzt, die bereits die zu vor beschriebene bevorzugte Form und Geometrie aufweisen.

Trägermaterialien bzw. Katalysatorvorläufer, die nicht die zuvor beschriebene, bevorzugte Formaufweisen, können einem Formgebungsschritt unterzogen werden.

Im Rahmen der Formgebung werden die Trägermaterialien bzw. Katalysatorvorläufer in der Regel konditioniert, in dem sie durch Vermahlen auf eine bestimmte Korngröße eingestellt werden.

Nach der Vermahlung kann das konditionierte Trägermaterial bzw. der konditionierte Katalysatorvorläufer mit weiteren Additiven, wie Formhilfsmitteln, beispielsweise Graphit, Bindemitteln, Porenbildner und Anteigmitteln vermischt werden und zu Formkörpern weiterverarbeitet werden. Vorzugsweise wird der Katalysatorvorläufer nur mit Graphit als Formhilfsmittel vermischt und keine weiteren Additive bei der Formgebung zugegeben.

Gängige Verfahren der Formgebung sind beispielsweise im Ullmann [Ullmann's Encyclopedia Electronic Release 2000, Kapitel: "Catalysis and Catalysts", Seiten 28-32] und von Ertl et al. [Ertl, Knözinger, Weitkamp, Handbook of Heterogenoeous Catalysis, VCH Weinheim, 1997, Seiten 98 ff] beschrieben.

Gängige Verfahren der Formgebung sind beispielsweise Extrusion, Tablettieren, d.h. mechanisches Verpressen oder Pelletieren, d.h. Kompaktieren durch kreisförmige und/oder rotierende Bewegungen.

Durch den Prozess der Formgebung können Formkörper mit der oben genannten Geometrie erhalten werden.

Die Formgebung kann aber auch durch Sprühtrocknung einer Suspension des Katalysatorvorläufers erfolgen.

Nach der Konditionierung bzw. Formgebung erfolgt in der Regel eine Temperung. Die Temperaturen bei der Temperung entsprechen üblicherweise den Temperaturen bei der Calcinierung.

Schritt b) Inkontaktbringen des in Schritt a) hergestellten Katalysatorvorläufers mit einer Ru/Co-Verbindung

### Inkontaktbringen mit einer löslichen Ru und Co-Verbindung

Erfindungsgemäß wird der Katalysatorvorläufer gleichzeitig oder nacheinander mit einer löslichen Ru-Verbindung und einer löslichen Co-Verbindung in Kontakt gebracht.

Der Ru-Gehalt der Lösungen, der mit dem Katalysatorvorläufer in Kontakt gebracht wird, liegt üblicherweise im Bereich von 0,1 bis 50 Gew.-%, bevorzugt 1 bis 40 Gew.-% und besonders bevorzugt 2 bis 15 Gew.-%.

Der Co-Gehalt der Lösungen, der mit dem Katalysatorvorläufer in Kontakt gebracht wird, liegt üblicherweise im Bereich von 0,1 bis 20 Gew.-%, bevorzugt 0,1 bis 5 Gew.-% und besonders bevorzugt 0,15 bis 2 Gew.-%.

Das Inkontaktbringen der Katalysatorvorläufer mit einer löslichen Ru-Verbindung bzw. einer löslichen Co-Verbindung erfolgt in der Regel im Anschluss an die Calcinierung des Katalysatorvorläufers oder im Anschluss an die Temperung nach dem Formgebungsschritt und vor der Reduktion/Passivierung des Katalysatorvorläufers

Das Inkontaktbringen des Katalysatorvorläufers mit einer löslichen Ru-Verbindung und einer löslichen Co-Verbindung erfolgt in der Regel durch Tränkung.

Die Katalysatorvorläufer, die bei der Tränkung verwendet werden, können beispielsweise in Form von Pulvern oder Formkörpern, wie Strängen, Zylindern,Tabletten, Kugeln oder Ringen, eingesetzt werden. Es werden bevorzugt Katalysatorvorläufer eingesetzt, die die voranstehend beschriebene Form und Geometrie aufweisen (siehe Abschnitt "Form und Geometrie der Trägermaterialien und Formkörper"). Besonders bevorzugt werden Katalysatorvorläufer in Form von Tabletten eingesetzt.

Die Höhe der Tabletten liegt bevorzugt im Bereich von 1 bis 10 und besonders bevorzugt im Bereich von 1,5 bis 3 mm. Das Verhältnis von Höhe h der Tablette zum Durchmesser D der Tablette beträgt bevorzugt, 1:1 bis 1:5, besonders bevorzugt 1:1 bis 2,5 und ganz besonders bevorzugt 1:1 bis 1:2.

Die Tränkung der Katalysatorvorläufer kann nach den üblichen Verfahren erfolgen (A. B. Stiles, Catalyst Manufacture - Laboratory and Commercial Preperations, Marcel Dekker, New York, 1983), beispielsweise durch Aufbringung eines löslichen Salzes von Ru und Co in einer oder mehreren Tränkstufen.

Als Salze von Ru bzw. Co kommen in der Regel wasserlösliche Salze, wie die Carbonate, Nitrate oder Nitrosylnitrate, Carboxylate, insbesondere die Nitrate oder Nitrosylnitrate, Acetate oder Chloride in Betracht.

Ganz besonders bevorzugt werden als Salze von Co und Ru die entsprechenden Nitrate oder Nitrosylnitrate eingesetzt, beispielsweise Cobaltnitrathexahydrat und Ru-nitrosylnitrat.

Die Tränkung der Katalysatorvorläufer kann auch nach der sogenannten "incipient wetness-Methode" erfolgen, bei der der Katalysatorvorläufer entsprechend seiner Wasseraufnahmekapazität maximal bis zur Sättigung mit der Tränklösung befeuchtet wird. Die Tränkung kann aber auch in überstehender Lösung erfolgen.

In einer bevorzugten Ausführungsform wird der Katalysatorvorläufer mit einer Lösung in Kontakt gebracht, die sowohl eine lösliche Verbindung von Ru als auch eine lösliche Verbindung von Co enthält.

In einer weiteren bevorzugten Ausführungsform wird der Katalysatorvorläufer in einer ersten Stufe mit einer Lösung in Kontakt gebracht, die eine lösliche Verbindung von Ru enthält und nachfolgend in einer zweiten Stufe mit einer Lösung in Kontakt gebracht, die eine lösliche Verbindungen von Co enthält.

In einer weiteren bevorzugten Ausführungsform wird der Katalysatorvorläufer in einer ersten Stufe mit einer Lösung in Kontakt gebracht, die eine lösliche Verbindung von Co enthält und nachfolgend in einer zweiten Stufe mit einer Lösung in Kontakt gebracht, die eine lösliche Verbindungen von Ru enthält.

Bei einstufigen und mehrstufigen Tränkverfahren wird der Katalysatorvorläufer nach den jeweiligen Tränkschritten vorzugsweise von der Tränklösung abgetrennt und getrocknet.

Optional kann nach dem jeweiligen Trocknungsschritt auch eine Calcinierung erfolgen. Es ist jedoch bevorzugt, dass nach dem jeweiligen Trocknungsschritt keine anschließende Calcinierung erfolgt.

Vorzugsweise wird der Katalysatorvorläufer im Anschluss an den letzten Trocknungsschritt; wie nachfolgend beschrieben. reduziert.

Durch das Inkontaktbringen des Katalysatorvorläufers mit den löslichen Verbindungen von Co und Ru
wird der Anteil von Ru im Katalysatorvorläufer um ca. 0,1 bis 5 Gew.-%, vorzugsweise 0,5 bis 4 Gew.-% und ganz besonders bevorzugt um 1 bis 3 Gew.-% erhöht und
der Anteil von Co im Katalysatorvorläufer um ca. 0,1 bis 5 Gew.-%, vorzugsweise 0,5 bis 3 Gew.-% und ganz besonders bevorzugt um 1 bis 2 Gew.-% erhöht, jeweils bezogen auf die Gesamtmasse des Katalysatorvorläufers.

Nach dem Inkontaktbringen des Katalysatorvoläufers mit den löslichen Verbindungen von Co und Ru enthält der Katalysatorvorläufer im Anschluss an die letzte Trocknung bevorzugt (wobei die Gewichtsangaben auf die Gesamtmasse des Katalysatorvorläufers bezogen sind)
0,1 bis 20 Gew.-%, besonders bevorzugt 0,5 bis 15 Gew.-% und insbesondere bevorzugt 1 bis10 Gew.-% katalytisch aktive Komponenten von Ru, berechnet als RuO₂, und
0,1 bis 50 Gew.-%, besonders bevorzugt 10 bis 45 Gew.-% und insbesondere bevorzugt 20 bis 40 Gew.%- katalytisch aktive Komponenten von Co, berechnet als CoO.

Nach dem Inkontaktbringen des Katalysatorvorläufers mit den löslichen Verbindungen von Ru und Co wird der Katalysatorvorläufer reduziert.

Vorzugsweise erfolgt die Reduktion im Anschluss an den letzten Tränkungsschritt nach dem Inkontaktbringen mit den löslichen Verbindungen von Ru und Co.

### Reduktion/Passivierung

Erfindungsgemäß erfolgt die Umsetzung von MEG und/oder MEA und Ammoniak an einem reduzierten Katalysatorvorläufer.

Durch die Reduktion wird der Katalysatorvorläufer in der Regel in seine katalytisch aktive Form umgewandelt.

Die Reduktion des Katalysatorvorläufers wird bevorzugt bei erhöhter Temperatur durchgeführt. Als Reduktionsmittel wird üblicherweise Wasserstoff oder ein Wasserstoff enthaltendes Gas eingesetzt.

Der Wasserstoff kommt im Allgemeinen technisch rein zum Einsatz. Der Wasserstoff kann auch in Form eines Wasserstoff enthaltendem Gases, d.h. in Beimengungen mit anderen Inertgasen, wie Stickstoff, Helium, Neon, Argon oder Kohlendioxid zum Einsatz kommen. In einer bevorzugten Ausführungsform wird Wasserstoff zusammen mit Stickstoff eingesetzt, wobei der Volumenanteil von Wasserstoff bevorzugt im Bereich von 1 bis 50, besonders bevorzugt 2,5 bis 30 und inbesondere bevorzugt 5 bis 25 Vol-% beträgt. Der Wasserstoffstrom kann auch als Kreisgas in die Reduktion zurückgeführt werden, ggf. vermischt mit Frisch-Wasserstoff und ggf. nach Entfernen von Wasser durch Kondensation.

Es ist weiterhin bevorzugt den Anteil von Wasserstoff im Gemisch mit Inertgas graduell oder stufenweise zu erhöhen, beispielsweise von 0 Vol.% Wasserstoff auf 50 Vol.% Wasserstoff. So kann beim Aufheizen der Volumenanteil von Wasserstoff 0 Vol.-% betragen und nach Erreichen der Reduktionstemperatur in ein oder mehreren Stufen oder graduell auf 50 Vol-% erhöht werden.

Die Reduktion wird bevorzugt in einem Muffelofen, einem Drehrohrofen, einem Bandkalzinierofen oder einem bewegten oder unbewegten Reduktionsofen durchgeführt.

Die Reduktion des Katalysatorvorläufers erfolgt weiterhin bevorzugt in einem Reaktor, in dem die Katalysatorvorläufer als Festbett angeordnet sind. Besonders bevorzugt erfolgt die Reduktion des Katalysatorvorläufers in demselben Reaktor in dem die nachfolgende Umsetzung von MEG und/oder MEA mit NH3 erfolgt.

Weiterhin kann die Reduktion des Katalysatorvorläufers in einem Wirbelschichtreaktor in der Wirbelschicht erfolgen.

Die Reduktion des Katalysatorvorläufers erfolgt in der Regel bei Reduktionstemperaturen von 50 bis 600°C, insbesondere von 100 bis 500°C, besonders bevorzugt von 150 bis 450°C und insbesondere bevorzugt 200 bis 300°C.

Der Wasserstoffpartialdruck beträgt in der Regel von 1 bis 300 bar, insbesondere von 1 bis 200 bar, besonders bevorzugt von 1 bis 100 bar, wobei sich die Druckangaben hier und im Folgenden auf den absolut gemessenen Druck beziehen.

Die Dauer der Reduktion ist im Allgemeinen abhängig von der Größe und Form des Reaktors und wird in der Regel nur so schnell durchgeführt, dass ein größerer Temperaturanstieg im Reaktor vermieden wird. Dies bedeutet, dass je nach Form und Größe des Reaktors die Reduktion von mehreren Stunden bis mehreren Wochen in Anspruch nehmen.

Während der Reduktion kann ein Lösungsmittel zugeführt werden, um entstehendes Reaktionswasser abzuführen und/oder um beispielsweise den Reaktor schneller aufheizen zu können und/oder während der Reduktion die Wärme besser abführen zu können. Das Lösungsmittel kann hierbei auch überkritisch zugeführt werden.

Geeignete Lösungsmittel können die zuvor beschriebenen Lösungsmittel eingesetzt werden. Bevorzugte Lösungsmittel sind Wasser; Ether wie Methyltertbutylether, Ethyltertbutylether, Dioxan oder Tetrahydrofuran. Besonders bevorzugt sind Wasser oder Tetrahydrofuran. Als geeignete Lösungsmittel kommen ebenfalls geeignete Mischungen in Betracht.

Der reduzierte Katalysator kann im Anschluss an die Reduktion direkt mit den Edukten, wie MEG, MEA und NH3, in Kontakt gebracht werden. Dies ist insbesondere Vorteilhaft, wenn die Reduktion in dem Reaktor erfolgt ist, in dem auch die nachfolgende Umsetzung von MEG und/oder MEA erfolgt.

Der so reduzierte Katalysator kann aber auch nach der Reduktion unter inerten Bedingungen gehandhabt werden. Bevorzugt kann der Katalysatorvorläufer unter einem Inertgas wie Stickstoff gehandhabt und gelagert werden oder unter einer inerten Flüssigkeit, zum Beispiel einem Alkohol, Wasser oder dem Produkt der jeweiligen Reaktion, für die der Katalysator eingesetzt wird. Gegebenenfalls muss der Katalysator vor Beginn der eigentlichen Reaktion dann von der inerten Flüssigkeit befreit werden. Die Lagerung des Katalysators unter inerten Substanzen ermöglicht eine unkomplizierte und ungefährliche Handhabung und Lagerung des Katalysators.

### Passivierung

Der Katalysator kann nach der Reduktion mit einem Sauerstoff enthaltenden Gasstrom, wie Luft oder einem Gemisch von Luft mit Stickstoff, in Kontakt gebracht werden.

Dadurch wird ein passivierter Katalysator erhalten. Der passivierte Katalysator weist im Allgemeinen eine schützende Oxidschicht auf. Durch diese schützende Oxidschicht wird die Handhabung und Lagerung des Katalysators vereinfacht, so dass beispielsweise der Einbau des passivierten Katalysators in den Reaktor vereinfacht wird.

Zur Passivierung wird im Anschluss an den Reduktionsschritt der reduzierte Katalysator mit einem sauerstoffhaltigen Gas, bevorzugt Luft, in Kontakt gebracht wird.

Das sauerstoffhaltige Gas kann in Beimengungen mit Inertgasen, wie Stickstoff, Helium, Neon, Argon oder Kohlendioxid zum Einsatz kommen. In einer bevorzugten Ausführungsform wird Luft zusammen mit Stickstoff eingesetzt, wobei der Volumenanteil von Luft bevorzugt im Bereich von 1 bis 80, besonders bevorzugt 20 bis 70 und insbesondere bevorzugt 30 bis 60 Vol-% beträgt. In einer bevorzugten Ausführungsform wird der Volumenanteil von Luft im Gemisch mit Stickstoff allmählich von 0 auf ungefähr 50 Vol.-% erhöht.

Die Passivierung erfolgt bevorzugt bei Temperaturen bis 50°C, bevorzugt bis 45°C und ganz besonders bevorzugt bis 35°C.

### Aktivierung

Ein passivierter Katalysator wird bevorzugt vor dem Inkontaktbringen mit den Edukten durch Behandlung des passivierten Katalysators mit Wasserstoff oder einem Wasserstoff enthaltenden Gas reduziert. Die Bedingungen bei der Aktivierung entsprechen im Allgemeinen den Reduktionsbedingungen, die bei der Reduktion angewandt werden. Durch die Aktivierung wird in der Regel die schützende Passivierungsschicht aufgehoben.

### Edukte

Die erfindungsgemäße Umsetzung von Ethylenglykol (EG) und/oder Monoethanolamin (MEA) und Ammoniak (NH₃) in Gegenwart der reduzierten bzw. aktivierten Aminierungskatalysatoren erfolgt erfindungsgemäß in der Flüssigphase.

### Ethylenglykol

Als Ethylenglykol wird bevorzugt technisches Ethylenglykol mit einer Reinheit von mindestens 98%, und ganz besonders bevorzugt Ethylenglykol mit einer Reinheit von mindestens 99% und ganz besonders bevorzugt von mindestens 99,5%.

Das in das Verfahren eingesetzte Ethylenglykol kann aus Ethylen hergestellt werden, welches aus petrochemischen Prozessen erhältlich ist. So wird in der Regel in einer ersten Stufe Ethen zu Ethylenoxid oxidiert, welches nachfolgend mit Wasser zu Ethylenglykol umgesetzt wird. Das erhaltene Ethylenoxid kann aber auch im sogenannten Omega-Prozess mit Carbondioxid zu Ethylencarbonat umgesetzt werden, welches anschließend mit Wasser zu Ethylenglykol zu hydrolisieren kann. Das Omega-Verfahren zeichnet sich durch eine höhere Selektivität für Ethylenglykol aus, da weniger Nebenprodukte, wie Di- und Triethylenglykol entstehen.

Ethen kann aber auch aus nachwachsen Rohstoffen hergestellt werden. So kann Ethen durch Dehydratisierung von Bio-Ethanol gebildet werden.

Ethylenglykol lässt sich auch über den Synthesegasweg, z.B. durch oxidative Carbonylierung von Methanol zu Dimethyloxalat und dessen anschließender Hydrierung, herstellen. Damit kommen als weiterer petrochemischer Rohstoff auch Erdgas oder Kohle für die Herstellung von MEG in Frage.

### MEA

In das erfindungsgemäße Verfahren kann auch MEA eingesetzt werden.

MEA kann, wie voranstehend beschrieben, durch Umsetzung von Ethylenoxid mit Ammoniak hergestellt werden.

Vorzugsweise kann MEA durch Umsetzung von MEG mit Ammoniak hergestellt werden, beispielsweise durch das erfindungsgemäße Verfahren, in dem zunächst MEG mit Ammoniak umgesetzt wird und das neben EDA entstehende MEA von EDA getrennt wird und das abgetrennte MEA, ggf. zusammen mit nicht umgesetzten MEG, in das erfindungsgemäße Herstellungsverfahren zurückführt wird.

Wenn MEA als ohne MEG in das erfindungsgemäße Verfahren eingesetzt wird, so wird MEA bevorzugt mit einer Reinheit von mindestens 97%, und ganz besonders bevorzugt mit einer Reinheit von mindestens 98% und ganz besonders bevorzugt von mindestens 99% eingesetzt. Wenn MEA zusammen mit MEG in das erfindungsgemäße Verfahren eingesetzt wird, so liegt der Gewichtsanteil von MEA in Bezug auf die Masse von MEA und MEG bevorzugt im Bereich von 0 bis 60 Gew.-%, besonders bevorzugt 10 bis 50 Gew.-% und ganz besonders bevorzugt 20 bis 40 Gew.-%.

### Ammoniak

Erfindungsgemäß erfolgt die Umsetzung von Ethylenglykol und/oder Monoethanolamin mit Ammoniak.

Als Ammoniak kann herkömmlich im Handel erhältliches Ammoniak eingesetzt werden, beispielsweise Ammoniak mit einem Gehalt von mehr 98 Gew.-% Ammoniak, bevorzugt mehr als 99 Gew.-% Ammoniak, bevorzugt mehr als 99,5 Gew.-%, insbesondere mehr als 99,8 Gew.-% Ammoniak.

### Wasserstoff

Das erfindungsgemäße Verfahren erfolgt bevorzugt in Gegenwart von Wasserstoff.

Der Wasserstoff kommt im Allgemeinen technisch rein zum Einsatz. Der Wasserstoff kann auch in Form eines Wasserstoffs enthaltenden Gases, d.h. mit Beimengungen anderer Inertgase, wie Stickstoff, Helium, Neon, Argon oder Kohlendioxid zum Einsatz kommen. Als Wasserstoff enthaltende Gase können beispielsweise Reformerabgase, Raffineriegase usw. verwendet werden, wenn und soweit diese Gase keine Kontaktgifte für die eingesetzten Katalysatoren, wie zum Beispiel CO enthalten. Bevorzugt wird jedoch reiner Wasserstoff bzw. im Wesentlichen reiner Wasserstoff in das Verfahren eingesetzt, beispielsweise Wasserstoff mit einem Gehalt von mehr als 99 Gew.-% Wasserstoff, bevorzugt mehr als 99,9 Gew.-% Wasserstoff, besonders bevorzugt mehr als 99,99 Gew.-% Wasserstoff, insbesondere mehr als 99,999 Gew.-% Wasserstoff.

### Umsetzung in der Flüssigphase

Erfindungsgemäß erfolgt die Umsetzung von Ethylenglykol mit Ammoniak und einem Aminierungskatalysator in der Flüssigphase.

Im Rahmen der vorliegenden Erfindung bedeutet Umsetzung in der Flüssigphase, dass die Reaktionsbedingungen, wie Druck und Temperatur, so eingestellt werden, dass Ethylenglykol in der Flüssigphase vorliegt und den Aminierungskatalysator flüssig umströmt.

Die Umsetzung von MEG und/oder mit Ammoniak kann kontinuierlich oder diskontinuierlich durchgeführt werden. Bevorzugt ist eine kontinuierliche Umsetzung.

### Reaktoren

Geeignete Reaktoren für die Reaktion in der Flüssgiphase sind im Allgemeinen Rohrreaktoren. In den Rohrreaktoren kann der Katalysator als Fließ- oder Festbett angeordnet sein. Besonders bevorzugt erfolgt die Umsetzung von Ethylenglykol und/oder Monoethanolamin mit NH₃ in einem Rohrreaktor, in dem der Aminierungskatalysator als Festbett angeordnet ist.

Ist der Katalysator als Festbett angeordnet, kann es für die Selektivität der Reaktion vorteilhaft sein, die Katalysatoren im Reaktor mit inerten Füllkörpern zu vermischen, sie sozusagen zu "verdünnen". Der Anteil der Füllkörper in solchen Katalysatorzubereitungen kann 20 bis 80, bevorzugt 30 bis 60 und besonders bevorzugt 40 bis 50 Volumenteile betragen.

Alternativ erfolgt die Umsetzung vorteilhaft in einem Rohrbündelreaktor oder in einer Monostranganlage.Bei einer Monostranganlage kann der Rohrreaktor, in dem die Umsetzung erfolgt, aus einer Hintereinanderschaltung mehrerer (z.B. zweier oder dreier) einzelner Rohrreaktoren betehen. Optional ist hier vorteilhaft eine Zwischeneinspeisung von Feed (enthaltend das Edukt und/oder Ammoniak und/oder H₂) und/oder Kreisgas und/oder Reaktraustrag aus einem nachgeschalteten Reaktor möglich.

### Reaktionsbedingungen

Beim Arbeiten in der Flüssigphase leitet man bevorzugt das MEG und/oder plus Ammoniak simultan in flüssiger Phase bei Drücken von im Allgemeinen 5 bis 30 MPa (50-300 bar), bevorzugt 5 bis 25 MPa, besonders bevorzugt 20 15 bis 25 MPa, und Temperaturen von im Allgemeinen 80 bis 350 °C, besonders 100 bis 300 °C, bevorzugt 120 bis 270 °C, besonders bevorzugt 130 bis 250 °C, insbesondere 160 bis 230 °C, inklusive Wasserstoff über den Katalysator, der sich üblicherweise in einem bevorzugt von außen beheizten Festbettreaktor befindet.

Der Wasserstoffpartialdruck beträgt vorzugsweise 0,25 bis 20 MPa (2,5 bis 200 bar), besonders bevorzugt 0,5 bis 15 MPa (5 bis 150 bar), ganz besonders bevorzugt 1 bis 10 MPa (10 bis 100 bar) und insbesondere bevorzugt 2 bis 5 MPa (20 bis 50 bar).

### Eintrag

MEG und/oder MEA und Ammoniak werden dem Reaktor bevorzugt in flüssiger Form zugeführt und in flüssiger Form mit dem Aminierungskatalysator in Kontakt gebracht.

Es ist dabei sowohl eine Rieselfahrweise als auch eine Sumpffahrweise möglich.

Es ist zweckmäßig, die Reaktanden bereits vor der Zuführung in das Reaktionsgefäß zu erwärmen, und zwar bevorzugt auf die Reaktionstemperatur.

Ammoniak wird bevorzugt in der 0,90- bis 100-fachen molaren Menge, insbesondere in der 1,0-bis 20-fachen molaren Menge, jeweils bezogen auf das eingesetzte MEG bzw. MEA eingesetzt. Die Katalysatorbelastung liegt im Allgemeinen im Bereich von 0,05 bis 5, bevorzugt 0, 1 bis 2, besonders bevorzugt 0,2 bis 0,6 kg (MEG+MEA) pro kg Katalysator und Stunde.

Die Bei den angegebenen Katalysatorbelastungen liegt der Umsatz von MEG bzw. MEA in der Regel im Bereich von 20 bis 75%, bevorzugt im Bereich von 30 bis 60% und ganz besonders bevorzugt im Bereich von 35 bis 55%.

Das im Zuge der Umsetzung gebildete Reaktionswasser jeweils ein Mol pro Mol umgesetzte Alkoholgruppe wirkt sich im Allgemeinen auf den Umsetzungsgrad, die Reaktionsgeschwindigkeit, die Selektivität und die Katalysatorstandzeit nicht störend aus und wird deshalb zweckmäßigerweise erst bei der Aufarbeitung des Reaktionsproduktes aus diesem entfernt, z. B. destillativ.

### Austrag

Der Austrag aus dem Aminierungsreaktor enthält die Produkte der Aminierungsreaktion, nicht umgesetzte Edukte, wie Ethylenglykok und Ammoniak, sowie Wasserstoff und Wasser.

Als Produkte der Aminierungsreaktion enthält der Austrag aus dem Aminierungsreaktor weiterhin die entsprechenden Ethanolamine und/oder Ethylenamine auf Basis von MEG.

Bevorzugt enthält der Austrag aus dem Aminierungsreaktor MEA und/oder EDA.

Als Produkte der Aminierungsreaktion enthält der Reaktionsaustrag weiterhin bevorzugt höhere, lineare Ethylenamine der allgemeinen Formel

R-CH₂-CH₂-NH₂,

wobei R ein Rest der Formel -(NH-CH₂-CH₂)_{X}-NH₂ ist, wobei x eine ganze Zahl im Bereich von 1 bis 4, bevorzugt 1 bis 3 uns ganz besonders bevorzugt 1 bis 2 ist. Bevorzugt enthält der Reaktionsaustrag, DETA , TETA und TEPA, ganz besonders bevorzugt DETA und TETA und insbesondere bevorzugt DETA.

Als Produkte der Aminierungsreaktion kann der Austrag aus dem Aminierungsreaktor auch höhere, lineare Ethanolamine der Formel

R-CH₂-CH₂-OH

enthalten, wobei R ein Rest der Formel -(NH-CH₂-CH₂)_{X}-NH₂ ist, wobei x eine ganze Zahl im Bereich von 1 bis 4, bevorzugt 1 bis 3 uns ganz besonders bevorzugt 1 bis 2 ist.

Ein Beispiel für ein höheres, lineares Ethanolamin ist AEEA.

Als Produkte der Aminierungsreaktion kann der Reaktionsaustrag kann auch zyklische Ethanolamine der Formel 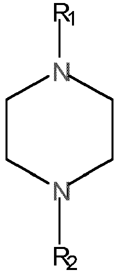 enthalten, wobei R₁ ein Rest der Formel -(CH₂-CH₂-NH)_{X}-CH₂-CH₂-OH ist, wobei x eine ganze Zahl im Bereich von 0 bis 4, bevorzugt 0 bis 3 und besonders bevorzugt 1 bis 2 ist, und
R₂ unabhängig oder gleichzeitiger entweder H oder ein Rest der Formel -(CH₂-CH₂-NH)_{X}-CH₂-CH₂-OH ist, wobei x eine ganze Zahl im Bereich von 0 bis 4, bevorzugt 0 bis 3 und besonders bevorzugt 1 bis 2 ist oder ein Rest der Formel -(CH₂-CH₂-NH)_{X}-CH₂-CH₂-NH₂ ist, wobei x eine ganze Zahl im Bereich von 0 bis 4, bevorzugt 0 bis 3 und besonders bevorzugt 1 bis 2 ist. Ein Beispiel für ein zyklisches Ethanolamin ist Hydroxyethylpiperazin (HEP).

Als Produkte der Aminierungsreaktion kann der Reaktionsaustrag kann auch zyklische Ethylenamine der allgemeinen Formel 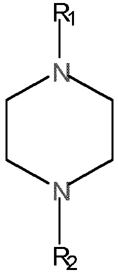 enthalten, wobei R₁ und R₂ unabhängig oder gleichzeitiger entweder H oder ein Rest der Formel -(CH₂-CH₂-NH)_{X}-CH₂-CH₂-NH₂ sein kann, wobei X eine ganze Zahl im Bereich von 0 bis 4, bevorzugt 0 bis 4 und besonders bevorzugt 1 bis 2 ist.

Beispiele für zyklische Ethylenamine, die im Reaktionsaustrag enthalten sind, sind Piperazin und AEPIP.

Bevorzugt enthält der Austrag 1 bis 60 Gew.-% MEA. 1 bis 90 Gew.-% EDA, 0,1 bis 30 Gew.-% höhere zyklische Ethylenamine, wie PIP und AEPIP, 0,1 bis 30 Gew.-% höhere lineare Ethylenamine, wie DETA, TETA und TEPA.

Besonders bevorzugt enthält der Austrag 10 bis 50 Gew.-% MEA. 25 bis 85 Gew.-% EDA, 0,25 bis 10 Gew.-% zyklische Ethylenamine, wie PIP und AEPIP, 1 bis 30 Gew.-% höhere lineare Ethylenamine, wie DETA, TETA und TEPA.

Ganz esonders bevorzugt enthält der Austrag 15 bis 45 Gew.-% MEA. 30 bis 70 Gew.-% EDA, 0,5 bis 5 Gew.-% zyklische Ethylenamine, wie PIP und AEPIP, 5 bis 25 Gew.-% höhere lineare Ethylenamine, wie DETA, TETA und TEPA.

Durch das erfindungsgemäße Verfahren können Selektivitätsquotienten SQ von 1,5 und mehr, bevorzugt 4 und mehr und besonders bevorzugt von 8 und mehr erreicht werden. Dies bedeutet, dass das Produktverhältnis von gewünschten linearen Ethylenaminen und Ethanolaminen, wie MEA und EDA zu nicht gewünschten zyklischen Ethylenaminen und nicht gewünschten höheren Ethanolaminen, wie PIP und AEEA, durch das erfindungsgemäße Verfahren erhöht werden kann.

Der Austrag wird in der Regel aufgearbeitet, so dass die verschiedenen Komponenten voneinander getrennt werden.

Dazu wird der Reaktionsaustrag zweckmäßiger Weise entspannt.

Die Komponenten, die nach der Entspannung in gasförmigen Form vorliegen, wie Wasserstoff und Inertgase, werden in der Regel in einem Gas-Flüssigabscheider von den flüssigen Komponenten getrennt. Die gasförmigen Komponenten können einzeln (nach einem weiteren Aufarbeitungsschritt) oder zusammen in den Aminierungsreaktor zurückgeführt werden.

Nach der Abtrennung von Wasserstoff und/oder Inertgas enthält der Austrag aus dem Aminerungsreaktor ggf. Ammoniak, nicht umgesetztes Ethylenglykol, Wasser sowie die Aminierungsprodukte.

Bevorzugt erfolgt die Auftrennung des Austrags aus dem Aminierungsreaktor in zwei Trennsequenzen, wobei jede Trennsequenz eine mehrstufige Destillation umfasst. Eine solche Aufarbeitung ist beispielsweise in der EP-B1-198699 beschrieben. Demgemäß werden in der ersten Trennsequenz werden zunächst Wasser und Ammoniak abgetrennt und in der zweiten Trennsequenz eine Auftrennung in nicht umgesetztes MEG, sowie MEA, EDA, PIP, DETA, AEEA und höhere Ethylenamine. Hierbei werden zunächst gegenüber dem Azeotrop von MEG und DETA niedriger sowie höher siedende Komponenten abgetrennt und anschließend das an MEG und DETA aufkonzentrierte Gemisch per Extraktivdestillation mit Triethylenglykol (TEG) als selektiven Lösungsmittel in einen MEG und DETA enthaltenden Strom aufgetrennt.

MEA kann teilweise oder vollständig, ggf. zusammen oder getrennt mit nicht umgesetztem MEG, in das erfindungsgemäße Verfahren zurückgeführt werden.

### Vorteile

In dem erfindungsgemäßen Verfahren ist es möglich MEG mit einer hohen Selektivtät für die linearen Aminierungsprodukte MEA und EDA umzusetzen, während die Selektivität für das zyklische Aminierungsprodukt PIP und das höhere Ethanolamin AEEA gering ist.

Ein Maß für diesen Effekt ist der Selektivitätsquotient SQ, der als Quotient aus der Summe der Selektivitäten von DETA und EDA und der Summe der Selektivitäten von PIP und AEEA (SQ=(S(DETA)+S(EDA))/(S(PIP)+S(AEEA)) definiert ist.

Die Erzielung eines hohen Selektivitätsquotienten SQ ist technisch vorteilhaft, da die Marktnachfrage nach den linearen Aminierungsprodukten MEA und EDA, sowie deren lineare Homologe, wie DETA und TETA, höher ist als die Nachfrage nach PIP bzw. AEEA.

Weiterhin werden durch das erfindungsgemäße Verfahren weniger unerwünschte Nebenprodukte gebildet. Unerwünschte Nebenprodukte sind beispielsweise gasförmige Zersetzungsprodukte oder unlösliche oder schwerlösliche Oligomere und Polymere auf Basis von MEA und EDA. Die Bildung solcher Nebenprodukte führt zu einer Verringerung der Kohlenstoffbilanz und zu damit zu einer Verringerung der Wirtschaftlichkeit des Verfahrens. Die Bildung von schwerlöslichen oder unlöslichen Nebenprodukte kann zu Ablagerung auf den Aminierungskatalysatoren führen, die die Aktivität der Aminierungskatalysatoren verringert.

Das erfindungsgemäße Verfahren führt ebenfalls zu einer Verringerung der Menge an N-Methylethylendiamin (NMEDA). NMEDA ist ein unerwünschtes Nebenprodukt. In vielen technischen Anwendungen wird eine Reinheit von EDA spezifiziert, bei der Anteil an NMEDA unter 500 Gew.-ppm liegt.

Weiterhin wurde festgestellt, dass die in das erfindungsgemäße Verfahren eingesetzten Katalysatorvorläufer in dem Verfahren eine hohe Aktivität aufweisen, so dass eine günstige Raum-Zeit-Ausbeute erzielt werden kann.

Insgesamt kann durch das erfindungsgemäße Verfahren ein vorteilhaftes Eigenschaftsspektrum in Bezug auf Gesamtselektivität, Selektivitätsquotient, Aktivität und die Bildung von unerwünschten Nebenprodukten erzielt werden.

Die Erfindung wird anhand der folgenden Beispiele erläutert:

### Herstellung der Katalysatorvorläufer

### Vergleichsbeispiel 1:

Der Katalysatorvorläufer wurde gemäß Beispiel B3 der WO 2013/072289 hergestellt. Vor der Reduktion der so hergestellten Tabletten, wurden diese zu 1-2 mm Splitt zerkleinert.

Der so erhaltene Katalysatorvorläufer wurde gemäß der folgenden Vorschrift reduziert (siehe Tabelle 1):

**Tabelle 1:**

| | Dauer (min) | Temperatur (°C) | Stickstoff (Nl/h) | Wasserstoff (Nl/h) | Luft (Nl/h) | Bemerkung |
|---|---|---|---|---|---|---|
| 1 | 30min | RT | 100 | - | - | Spülvorgang bei RT |
| 2 | 44min | 220 | 95 | 5 | - | Aufheizen auf 220°C |
| 3 | 120min | 220 | 95 | 5 | - | Haltezeit bei 220°C |
| 4 | 30min | 280 | 95 | 5 | - | Aufheizen auf 280°C |
| 5 | 15min | 280 | 95 | 5 | - | Erhöhung der Wasserstoffmenge |
| 6 | 15min | 280 | 90 | 10 | - | Erhöhung der Wasserstoffmenge |
| 7 | 15min | 280 | 80 | 20 | | Erhöhung der Wasserstoffmenge |
| 8 | 15min | 280 | 70 | 30 | | Erhöhung der Wasserstoffmenge |
| 9 | 15min | 280 | 60 | 40 | | Erhöhung der Wasserstoffmenge |
| 10 | 15min | 280 | 50 | 50 | | Abkühlvorgang auf RT |
| 11 | 120min | 280 | 50 | 50 | | Haltezeit bei 280°C |

Im Anschluss an die Reduktion erfolgte eine Passivierung des Katalysatorvorläufers. Hierzu wurde der reduzierte Katalysatorvorläufer mit einem Strom aus 50 NL/h N2 und 0 NL/h Luft umströmt. Die Luftmenge wurde langsam erhöht, während die N2-Menge langsam verringert wurde, bis 20 NL/h N2 und 20 NL/h Luft erreicht sind. Die Luftmengenerhöhung wurde so durchgeführt, dass die Katalysatortemperatur 35 °C nicht überstieg.

### Vergleichsbeispiel 2:

8,73 g Cobaltnitrathexahydrat (20,25 Gew.-% Co) und 1,85 g Nickel-Nitrathexahydrat (19 Gew.-% Ni) wurden vorgelegt.

Zu dem Gemisch wurden 56,85 g Ru-nitrosylnitratlösung (16 Gew.-% Ru) dazugeben. Die so erhaltene Lösung wurde mit VE-Wasser auf insgesamt 74 ml aufgefüllt.

Die so erhaltene Metallsalzlösung wurde in ein Sprühgefäß überführt.

150 g Al203-Träger (1-2 mm Splitt) wurden bei 900°C unter Luftatmosphäre calciniert. Im Anschluss wurde die maximale Wasseraufnahme bestimmt. Diese betrug 0,55 ml/g.

Der Katalysatorträger wurde auf einem Drehteller auf 90% der Wasseraufnahme mit der zuvor hergestellten Metallsalzlösung getränkt, in dem der Splitt auf dem Drehteller mit der entsprechenden Menge der Metallsalzlösung besprüht wurde.

Der mit der Metallsalzlösung getränkte Splitt wurde anschließend 16 h bei 120°C im Umlufttrockenschrank getrocknet.

Im Anschluss an die Trocknung wurde der Katalysatorvorläufer gemäß den in Tabelle 2, genannten Bedingungen reduktiv calciniert.

**Tabelle 2:**

| | Dauer (min) | Temperatur (°C) | Aufheizrate (°C/min) | Stickstoff (Nl/h) | Wasserstoff (Nl/h) | Luft (Nl/h) | Bemerkung |
|---|---|---|---|---|---|---|---|
| 1 | 30min | RT | Ohne | 100 | - | - | Spülvorgang bei RT |
| 2 | 150min | 150 | 1 | 95 | 5 | - | Aufheizen auf 150°C |
| 3 | 120min | 150 | Ohne | 95 | 5 | - | Haltezeit bei 150°C |
| 4 | 50min | | 1 | 95 | 5 | - | Aufheizen auf 150°C |
| 5 | 15min | 200 | Ohne | 95 | 5 | - | Erhöhung der Wasserstoffmenge |
| 6 | 15min | 200 | Ohne | 90 | 10 | - | Erhöhung der Wasserstoffmenge |
| 7 | 15min | 200 | Ohne | 80 | 20 | | Erhöhung der Wasserstoffmenge |
| 8 | 15min | 200 | Ohne | 70 | 30 | | Erhöhung der Wasserstoffmenge |
| 9 | 15min | 200 | Ohne | 60 | 40 | | Erhöhung der |
| | | | | | | | Wasserstoffmenge |
| 10 | 15min | 200 | Ohne | 50 | 50 | | Abkühlvorgang auf RT |
| 11 | 120min | 200 | Ohne | 50 | 50 | | Haltezeit bei 200°C |

Im Anschluss and die reduktive Kalzinierung wurde der Katalysator passiviert, in dem bei Raumtemperatur der Katalysator mit einem Gasstrom von 50 NL/h N2 und 0 NL/h Luft umströmt wurde. Die Luftmenge wurde langsam erhöht, während die N2-Menge langsam verringert wurde, bis 20 NL/h N2 und 20 NL/h Luft erreicht wurden. Die Luftmengenerhöhung wurde so durchgeführt, dass die Katalysatortemperatur 35°C nicht überstieg

### Vergleichsbeispiel 3:

8,73 g Cobaltnitrathexahydrat (20,25 Gew.-% Co) und 1,45 g Kupfer-Nitrathydrat (26,3 Gew.-% Cu) wurden vorgelegt.

Zu dem Gemisch wurden 56,85 g Ru-nitrosylnitratlösung (16 Gew.-% Ru) dazugeben. Die so erhaltene Lösung wurde mit VE-Wasser auf insgesamt 74 ml aufgefüllt.

Die so erhaltene Metallsalzlösung wurde in ein Sprühgefäß überführt.

150 g Al2O3-Träger (1-2 mm Splitt) wurden bei 900°C unter Luftatmosphäre calciniert. Im Anschluss wurde die maximale Wasseraufnahme bestimmt. Diese betrug 0,55 ml/g.

Der Katalysatorträger wurde auf einem Drehteller auf 90% der Wasseraufnahme mit der zuvor hergestellten Metallsalzlösung getränkt, in dem der Splitt auf dem Drehteller mit der Metallsalzlösung besprüht wurde.

Der mit der Metallsalzlösung getränkte Splitt wurde anschließend 16 h bei 120°C im Umlufttrockenschrank getrocknet.

Im Anschluss an die Trocknung wurde der Katalysatorvorläufer wie in Vergleichsbeispiel 2 reduktiv calciniert und passiviert.

### Beispiel 1:

Ein Katalysatorvorläufer wurde gemäß Beispiel B3 der WO 2013/072289 hergestellt.

Die so erhaltenen Tabletten (3*3 mm) wurden zu 1-2 mm Splitt zerkleinert. Die maximale Wasseraufnahmekapazität des Splitts betrug 0,30 mL/g.

Es wurde eine Metallsalzlösung hergestellt. Hierzu wurden 20,25 g Cobaltnitrathexahydrat (20,25 Gew.-% Co) in heißem Wasser gelöst und 37,91 g Ru-nitrosylnitratlösung zugegeben. Die so erhaltene Lösung wurde mit VE-Wasser auf 71 ml aufgefüllt und in ein Sprühgefäß überführt.

Der Splitt wurde in einer Tränkapparatur mit einer Menge besprüht, die 95% der maximalen Wasseraufnahme des Splitts entspricht. Um die homogene Aufnahme der Tränklösung zu gewährleisten, wurde der Splitt noch weitere 30 min nachrotiert.

Im Anschluss wurde der Katalysatorsplitt für 16 h bei 120°C im Umlufttrockenschrank getrocknet.

Der so erhaltene Katalysatorvorläufer wurde wie in Vergleichsbeispiel 2 beschrieben reduktiv calciniert und passiviert.

### Katalysatortestung:

Die Testung der Katalysatoren erfolgte in einer kontinuierlich betriebenen Mehrfachanlage im Technikumsmaßstab. Der Reaktionsteil der Anlage besteht aus acht Einzelreaktoren, von denen jeweils vier in einem Reaktorblock (Heizblock) zusammengefasst sind. Jeder Einzelreaktor ist ein 1,5 m langes Edelstahlrohr mit einem Innendurchmesser von 8 mm. Die Rohre sind in einem elektrisch beheizten Reaktorblock bestehend aus einer Al-Mg-Legierung eingebaut.

Der Katalysator wurde in Form von Splitt (1,5 mm - 2 mm) in den Reaktor eingefüllt und auf einer ca. 33 cm langen Inertschüttung bestehend aus 3 mm großen Glasperlen gelagert. Oberhalb des Katalysatorbetts schließt sich eine weitere, 15 cm lange Inertschüttung bestehend aus 3 mm großen Glasperlen an.

Der Katalysator und die Inertschüttung wurden im Reaktor durch einen 1 cm langen Gewebedraht fixiert.

Jeder Reaktor wurde im geraden Durchgang betrieben und von unten angeströmt.

Das flüssige Edukt wurde aus einer Vorlage mit Hilfe einer HPLC-Pumpe zugeführt. Die Zuführung von Wasserstoff, Stickstoff und Ammoniak erfolgte durch separate Rohrleitungen.

Proben der flüssigen Reaktorausträge wurden hinter dem Reaktorausgang aus einem Abscheider entnommen. Die Reaktorausträge wurden gaschromatographisch analysiert.

Die Aktivierung des Katalysators erfolgte vor der Reaktion bei 200 °C und 170 bar über eine Zeit von 18 h in einem 50:50-Gemisch aus Wasserstoff und Stickstoff.

Die Testung aller Katalysatoren erfolgte unter folgenden Bedingungen:
- Temperatur: 165 °C
- Druck: 170 bar
- H2: 5 NL/h
- N2: 10 NL/h
- Molares Verhältnis NH3 : MEG = 10 : 1
- Katalysatorbelastung: 0,3 kg/L/h - 0,5 kg/L/h
- Katalysatorvolumen: 50 mL

Die genauen Bedingungen sind in der nachfolgenden Tabelle 3 zusammengefasst.

**Tabelle 3:**

| Katalysator | Kat.belastung /kg/L/h | Umsatz / FI.-% | EDA / FI.-% | DETA / FI.-% | AEEA / FI.-% | PIP / Fl.-% | MEA / Fl.-% | NMEDA + NEEDA + EtNH2 / FI.-% | Ges.Sel. (5 Hauptprodukte) / FI.-% | (EDA+DETA) /(PIP+AEEA) |
|---|---|---|---|---|---|---|---|---|---|---|
| Vergleichsbsp. 1 | 0,3 | 27,0 | 11,6 | 0,9 | 0,9 | 1,6 | 11,4 | 0,0 | 97,9 | 5,0 |
| Vergleichsbsp. 2 | 0,3 | 18,5 | 10,3 | 0,4 | 0,2 | 0,4 | 6,7 | 0,3 | 97,1 | 17,9 |
| Vergleichsbsp. 3 | 0,3 | 12,4 | 7,0 | 0,1 | 0,1 | 0,2 | 4,9 | 0,1 | 98,3 | 30,6 |
| Beispiel 1 | 0,3 | 36,4 | 14,0 | 2,7 | 2,0 | 4,7 | 11,3 | 0,1 | 95,1 | 2,5 |

Vergleichsbeispiel 1 zeigt einen Katalysator, der die Aktivmetalle Ni, Co, Cu und Sn enthält.

Beispiel 1 unterscheidet sich von Vergleichsbeispiel 1 darin, dass der Katalysator aus Vergleichsbeispiel 1 mit Co und Ru nachgetränkt wurde. Es ist ersichtlich, dass durch die Nachtränkung die Aktivität deutlich erhöht werden konnte.

In Vergleichsbeispiel 2 und 3 wurden Katalysatoren, die die Kombination von Ru, Co und Ni bzw. Ru, Co und Cu enthalten, direkt durch Tränkung von löslichen Verbindungen von Ru, Co und Ni bzw. Cu auf einem Katalysatorträger aus Aluminiumoxid hergestellt.

In Beispiel 1 wurde ein Ni-haltiger Katalysatorvorläufer, der durch Auffällung von Ni, Cu, Sn und Co auf ein Trägermaterial aus Aluminiumoxid hergestellt wurde, mit Ru und Co nachgetränkt.

Die Vergleichsbeispiele, die direkt durch Tränkung von Trägermaterialien mit den entsprechenden Aktivmetallen erhalten wurden, zeigen zwar eine hohe Selektivität und eine geringe Bildung von unerwünschten Nebenprodukten, wie NMEDA, aber eine wesentlich geringere Aktivität.

Nur mit Katalysatorvorläufern, die mit Ru und Co nachgetränkt wurden, kann ein ausgewogenes Eigenschaftsprofil in Bezug auf Aktivität, Selektivität und der Bildung von unerwünschten Nebenprodukten erreicht werden.

## Patentansprüche

1. Verfahren zur Herstellung von Alkanolaminen und/oder Ethylenaminen in der Flüssigphase, durch Umsetzung von Ethylenglykol und/oder Monoethanolamin mit Ammoniak in Gegenwart eines Aminierungskatalysators, welcher durch Reduktion eines Katalysatorvorläufers erhalten wird, **dadurch gekennzeichnet, dass** die Herstellung des Katalysatorvorläufers einen Schritt a) umfasst, in dem zunächst ein Katalysatorvorläufer hegestellt wird der ein oder mehrere katalytisch aktive Komponenten von Sn, Cu und Ni enthält und der in Schritt a) hergestellte Katalysatorvorläufer in einem Schritt b) gleichzeitig oder nacheinander mit einerlöslichen Ru-Verbindung und einer löslichen Co-Verbindung in Kontakt gebracht wird, und worin der Katalysatorvorläufer, der in Schritt a) hergestellt wird, zusätzlich katalytisch aktive Komponenten von Co enthält.

2. Verfahren nach m Anspruch 1, **dadurch gekennzeichnet, dass** der Katalysatorvorläufer in Schritt a) durch Mischfällung hergestellt wird und vor dem Inkontaktbringen mit Ru und Co in Schritt b) im Bereich von 1 bis 95 Gew.-% katalytisch aktive Komponenten von Sn, Cu und/oder Ni enthält, berechnet als CuO, NiO bzw SnO und jeweils bezogen auf die Gesamtmasse des Katalysatorvorläufers.

3. Verfahren nach Anspruch 1, **dadurch gekennzeichnet, dass** der Katalysatorvorläufer in Schritt a) durch Auffällung hergestellt wird und vor dem Inkontaktbringen mit Ru und Co in Schritt b) im Bereich von 5 bis 95 Gew.-% Trägermaterial und im Bereich von von 5 bis 90 Gew.-% katalytisch aktive Komponenten Sn, Cu und/oder Ni enthält, berechnet als CuO, NiO bzw SnO und jeweils bezogen auf die Gesamtmasse des Katalysatorvorläufers.

4. Verfahren nach Anspruch 1, **dadurch gekennzeichnet, dass** der Katalysatorvorläufer in Schritt a) durch Tränkung hergestellt wird und vor dem Inkontaktbringen mit Ru und Co in Schritt b) im Bereich von 50 bis 99 Gew.-% Trägermaterial und im Bereich von von 1 bis 50 Gew.-% katalytisch aktive Komponenten von Sn, Cu und/oder Ni enthält, berechnet als CuO, NiO bzw SnO und jeweils bezogen auf die Gesamtmasse des Katalysatorvorläufers.

5. Verfahren Anspruch 1, **dadurch gekennzeichnet, dass** der in Schritt a) hergestellte Katalysatorvorläufer
- 10 bis 75 Gew.-% katalytisch aktive Komponenten des Zirkoniums, berechnet als ZrO₂;
- 1 bis 30 Gew.-% katalytisch aktive Komponenten des Kupfers, berechnet als CuO,
- 10 bis 70 Gew.-% katalytisch aktive Komponenten des Nickels, berechnet als NiO,
- 0,1 bis 10 Gew.-% katalytisch aktive Komponenten eines oder mehrerer Metalle, ausgewählt aus Sb, Pb, Bi und In, jeweils berechnet als Sb₂O₃, PbO, Bi₂O₃ bzw. In₂O₃.
enthält, bezogen auf die Gesamtmasse des Katalysatorvorläufers.

6. Verfahren nach Anspruche 1, **dadurch gekennzeichnet, dass** der in Schritt a) hergestellte Katalysatorvorläufer
- 10 bis 75 Gew.-% katalytisch aktive Komponenten des Zirkoniums, berechnet als ZrO₂,
- 1 bis 30 Gew.-% katalytisch aktive Komponenten des Kupfers, berechnet als CuO,
- 10 bis 70 Gew.-% katalytisch aktive Komponenten des Nickels, berechnet als NiO,
- 10 bis 50 Gew.-% katalytisch aktive Komponenten des Kobalts, berechnet als CoO, und
- 0,1 bis 10 Gew.% katalytisch aktive Komponenten eines oder mehrerer Metalle, ausgewählt aus Pb, Bi, Sn, Sb und In, jeweils berechnet als PbO, Bi₂O₃, SnO, Sb₂O₃ bzw. In₂O₃
enthält, bezogen auf die Gesamtmasse des Katalysatorvorläufers.

7. Verfahren nach Anspruch 1, **dadurch gekennzeichnet, dass** der in Schritt a) hergestellte Katalysatorvorläufer
- 20 bis 70 Gew.-% katalytisch aktive Komponenten des Zirkoniums, berechnet als ZrO₂,
- 15 bis 60 Gew.-% katalytisch aktive Komponenten des Nickels, berechnet als NiO,
- 0,5 bis 14 Gew.% katalytisch aktive Komponenten des Eisens, berechnet als Fe₂O₃, und
- 0,2 bis 5,5 Gew.-%, katalytisch aktive Komponenten des Zinns, Bleis, Bismuths, Molybdäns, Antimons und/oder Phosphors, jeweils berechnet als SnO, PbO, Bi₂O₃, MoO₃, Sb₂O₃ bzw. H₃PO₄,
enthält, bezogen auf die Gesamtmasse des Katalysatorvorläufers.

8. Verfahren nach Anspruch 1, **dadurch gekennzeichnet, dass** der in Schritt a) hergestellt Katalysatorvorläufer
- 20 bis 85 Gew.-% katalytisch aktive Komponenten des Zirkoniums, berechnet als ZrO₂,
- 0,2 bis 25 Gew.-% katalytisch aktive Komponenten des Kupfers, berechnet als CuO,
- 0,2 bis 45 Gew.-% katalytisch aktive Komponenten des Nickels, berechnet als NiO,
- 0,2 bis 40 Gew.-% katalytisch aktive Komponenten des Kobalts, berechnet als CoO,
- 0, 1 bis 5 Gew.-% katalytisch aktive Komponenten des Eisens, berechnet als Fe₂O₃, und
- 0, 1 bis 5,0 Gew.-% katalytisch aktive Komponenten des Bleis, Zinns, Bismuths und/oder Antimons, jeweils berechnet als PbO, SnO, Bi₂O₃ bzw. Sb₂O₃
enthält, bezogen auf die Gesamtmasse des Katalysatorvorläufers.

9. Verfahren nach Anspruch 1, **dadurch gekennzeichnet, dass** der in Schritt a) hergestellte Katalysatorvorläufer
- 46 bis 65 Gew.-% katalytisch aktive Komponenten des Zirkoniums, berechnet als ZrO₂,
- 5,5 bis 18 Gew.-% katalytisch aktive Komponenten des Kupfers, berechnet als CuO,
- 20 bis 45 Gew.-% katalytisch aktive Komponenten des Nickels, berechnet als NiO,
- 1,0 bis 5,0 Gew.-% katalytisch aktive Komponenten des Kobalts, berechnet als CoO, und
- 0,2 bis 5,0 Gew.-% katalytisch aktive Komponenten des Vanadiums, Niobs, Schwefels, Phosphors, Galiums, Bors, Wolframs, Bleis und/oder Antimons, jeweils berechnet als V₂O₅, Nb₂O₅, H₂SO₄, H₃PO₄, Ga₂O₃, B₂O₃, WO₃, PbO bzw. Sb₂O₃
enthält, bezogen auf die Gesamtmasse des Katalysatorvorläufers.

10. Verfahren nach Anspruch 1, **dadurch gekennzeichnet, dass** der in Schritt a) hergestellte Katalysatorvorläufer
- 0,2 bis 5,0 Gew.% katalytisch aktive Komponenten des Zinns, berechnet als SnO,
- 10 bis 30 Gew.-% katalytisch aktive Komponenten des Kobalts, berechnet als CoO,
- 15 bis 80 Gew.-% katalytisch aktive Komponenten des Aluminiums, berechnet als Al₂O₃,
- 1 bis 20 Gew.-% katalytisch aktive Komponenten des Kupfers, berechnet als CuO,
- 5 bis 35 Gew.-% katalytisch aktive Komponenten des Nickels, berechnet als NiO, und
- 0,2 bis 5,0 Gew.-% katalytisch aktive Komponenten des Yttriums, Lanthans, Cers und/oder Hafniums, jeweils berechnet als Y₂O₃, La₂O₃, Ce₂O₃ bzw. Hf₂O₃
enthält, bezogen auf die Gesamtmasse des Katalysatorvorläufers.

11. Verfahren nach Anspruch 1, **dadurch gekennzeichnet, dass** der in Schritt a) hergestellte Katalysatorvorläufer
- 0,2 bis 5 Gew.-% katalytisch aktive Komponenten des Zinn, berechnet als SnO
- 15 bis 80 Gew.-% katalytisch aktive Komponenten des Aluminium, berechnet als Al₂O₃
- 1 bis 20 Gew.-% katalytisch aktive Komponenten des Kupfer, berechnet als CuO
- 5 bis 35 Gew.-% katalytisch aktive Komponenten des Nickel, berechnet als NiO, und
- 5 bis 35 Gew.-% katalytisch aktive Komponenten des Cobalt, berechnet als CoO
enthält, bezogen auf die Gesamtmasse des Katalysatorvorläufers.

12. Verfahren nach Anspruch 11, **dadurch gekennzeichnet, dass** die Herstellung des Katalysatorvorläufers in Gegenwart von Zinnnitrat und einem Komplexbildner erfolgt.

13. Verfahren nach mindestens einem der Ansprüche 1 bis 12, **dadurch gekennzeichnet, dass** der Katalysatorvorläufer in Schritt b) gleichzeitig mit der löslichen Ru-Verbindung und der löslichen Co-Verbindung in Kontakt gebracht wird.

14. Verfahren nach mindestens einem der Ansprüche 1 bis 13, **dadurch gekennzeichnet, dass** die Konzentration der löslichen Ru-Verbindung mit der der in Schritt a) hergestellte Katalysatorvorläufer in Schritt b) in Kontakt gebracht wird, im Bereich von 0,1 bis 50 Gew.-% liegt und die Konzentration der löslichen Co-Verbindung mit der der Katalysatorvorläufer in Schritt b) in Kontakt gebracht wird im Bereich von 0,1 bis 20 Gew.-% liegt.

15. Verfahren nach mindestens einem der Ansprüche 1 bis 14, **dadurch gekennzeichnet, dass** die die Umsetzung von Ethylenglykol und/oder Monoethanolamin mit Ammoniak in der Flüssigphase bei einem Druck von 5 bis 30 MPa und einer Temperatur im Bereich von 80 bis 350 °C erfolgt.

## Claims

1. A process for preparing alkanolamines and/or ethyleneamines in the liquid phase, by reacting ethylene glycol and/or monoethanolamine with ammonia in the presence of an amination catalyst which is obtained by reducing a catalyst precursor, wherein the preparation of the catalyst precursor comprises a step a) in which a catalyst precursor comprising one or more catalytically active components of Sn, Cu and Ni is first prepared and the catalyst precursor prepared in step a) is contacted simultaneously or successively with a soluble Ru compound and a soluble Co compound in a step b), and wherein the catalyst precursor which is prepared in step a) additionally comprises catalytically active components of Co.

2. The process according to claim 1, wherein the catalyst precursor is prepared by coprecipitation in step a) and, before being contacted with Ru and Co in step b), comprises in the range from 1% to 95% by weight of catalytically active components of Sn, Cu and/or Ni, calculated as CuO, NiO and SnO respectively and based in each case on the total mass of the catalyst precursor.

3. The process according to claim 1, wherein the catalyst precursor is prepared by precipitative application in step a) and, before being contacted with Ru and Co in step b), comprises in the range from 5% to 95% by weight of support material and in the range from 5% to 90% by weight of catalytically active components of Sn, Cu and/or Ni, calculated as CuO, NiO and SnO respectively and based in each case on the total mass of the catalyst precursor.

4. The process according to claim 1, wherein the catalyst precursor is prepared by impregnation in step a) and, before being contacted with Ru and Co in step b), comprises in the range from 50% to 99% by weight of support material and in the range from 1% to 50% by weight of catalytically active components of Sn, Cu and/or Ni, calculated as CuO, NiO and SnO respectively and based in each case on the total mass of the catalyst precursor.

5. The process according to claim 1, wherein the catalyst precursor prepared in step a) comprises
- 10% to 75% by weight of catalytically active components of zirconium, calculated as ZrO₂;
- 1% to 30% by weight of catalytically active components of copper, calculated as CuO,
- 10% to 70% by weight of catalytically active components of nickel, calculated as NiO,
- 0.1% to 10% by weight of catalytically active components of one or more metals selected from Sb, Pb, Bi and In, each calculated as Sb₂O₃, PbO, Bi₂O₃ and In₂O₃ respectively,
based on the total mass of the catalyst precursor.

6. The process according to claim 1, wherein the catalyst precursor prepared in step a) comprises
- 10% to 75% by weight of catalytically active components of zirconium, calculated as ZrO₂,
- 1% to 30% by weight of catalytically active components of copper, calculated as CuO,
- 10% to 70% by weight of catalytically active components of nickel, calculated as NiO,
- 10% to 50% by weight of catalytically active components of cobalt, calculated as CoO, and
- 0.1% to 10% by weight of catalytically active components of one or more metals selected from Pb, Bi, Sn, Sb and In, each calculated as PbO, Bi₂O₃, SnO, Sb₂O₃ and In₂O₃ respectively,
based on the total mass of the catalyst precursor.

7. The process according to claim 1, wherein the catalyst precursor prepared in step a) comprises
- 20% to 70% by weight of catalytically active components of zirconium, calculated as ZrO₂,
- 15% to 60% by weight of catalytically active components of nickel, calculated as NiO,
- 0.5% to 14% by weight of catalytically active components of iron, calculated as Fe₂O₃, and
- 0.2% to 5.5% by weight of catalytically active components of tin, lead, bismuth, molybdenum, antimony and/or phosphorus, each calculated as SnO, PbO, Bi₂O₃, MoO₃, Sb₂O₃ and H₃PO₄ respectively,
based on the total mass of the catalyst precursor.

8. The process according to claim 1, wherein the catalyst precursor prepared in step a) comprises
- 20% to 85% by weight of catalytically active components of zirconium, calculated as ZrO₂,
- 0.2% to 25% by weight of catalytically active components of copper, calculated as CuO,
- 0.2% to 45% by weight of catalytically active components of nickel, calculated as NiO,
- 0.2% to 40% by weight of catalytically active components of cobalt, calculated as CoO,
- 0.1% to 5% by weight of catalytically active components of iron, calculated as Fe₂O₃, and
- 0.1% to 5.0% by weight of catalytically active components of lead, tin, bismuth and/or antimony, each calculated as PbO, SnO, Bi₂O₃ and Sb₂O₃ respectively,
based on the total mass of the catalyst precursor.

9. The process according to claim 1, wherein the catalyst precursor prepared in step a) comprises
- 46% to 65% by weight of catalytically active components of zirconium, calculated as ZrO₂,
- 5.5% to 18% by weight of catalytically active components of copper, calculated as CuO,
- 20% to 45% by weight of catalytically active components of nickel, calculated as NiO,
- 1.0% to 5.0% by weight of catalytically active components of cobalt, calculated as CoO, and
- 0.2% to 5.0% by weight of catalytically active components of vanadium, niobium, sulfur, phosphorus, gallium, boron, tungsten, lead and/or antimony, each calculated as V₂O₅, Nb₂O₅, H₂SO₄, H₃PO₄, Ga₂O₃, B₂O₃, WO₃, PbO and Sb₂O₃ respectively,
based on the total mass of the catalyst precursor.

10. The process according to claim 1, wherein the catalyst precursor prepared in step a) comprises
- 0.2% to 5.0% by weight of catalytically active components of tin, calculated as SnO,
- 10% to 30% by weight of catalytically active components of cobalt, calculated as CoO,
- 15% to 80% by weight of catalytically active components of aluminum, calculated as Al₂O₃,
- 1% to 20% by weight of catalytically active components of copper, calculated as CuO,
- 5% to 35% by weight of catalytically active components of nickel, calculated as NiO, and
- 0.2% to 5.0% by weight of catalytically active components of yttrium, lanthanum, cerium and/or hafnium, each calculated as Y₂O₃, La₂O₃, Ce₂O₃ and Hf₂O₃ respectively,
based on the total mass of the catalyst precursor.

11. The process according to claim 1, wherein the catalyst precursor prepared in step a) comprises
- 0.2% to 5% by weight of catalytically active components of tin, calculated as SnO,
- 15% to 80% by weight of catalytically active components of aluminum, calculated as Al₂O₃,
- 1% to 20% by weight of catalytically active components of copper, calculated as CuO,
- 5% to 35% by weight of catalytically active components of nickel, calculated as NiO, and
- 5% to 35% by weight of catalytically active components of cobalt, calculated as CoO,
based on the total mass of the catalyst precursor.

12. The process according to claim 11, wherein the catalyst precursor is prepared in the presence of tin nitrate and a complexing agent.

13. The process according to at least one of claims 1 to 12, wherein the catalyst precursor in step b) is simultaneously contacted with the soluble Ru compound and the soluble Co compound.

14. The process according to at least one of claims 1 to 13, wherein the concentration of the soluble Ru compound with which the catalyst precursor prepared in step a) is contacted in step b) is in the range from 0.1% to 50% by weight and the concentration of the soluble Co compound with which the catalyst precursor is contacted in step b) is in the range from 0.1% to 20% by weight.

15. The process according to at least one of claims 1 to 14, wherein the reaction of ethylene glycol and/or monoethanolamine with ammonia is effected in the liquid phase at a pressure of 5 to 30 MPa and a temperature in the range from 80 to 350°C.

## Revendications

1. Procédé de fabrication d'alcanolamines et/ou d'éthylène-amines en phase liquide, par réaction d'éthylèneglycol et/ou de monoéthanolamine avec de l'ammoniac en présence d'un catalyseur d'amination, lequel est obtenu par réduction d'un précurseur de catalyseur, **caractérisé en ce que** la fabrication du précurseur de catalyseur comprend une étape a), dans laquelle on fabrique d'abord un précurseur de catalyseur, qui contient un ou plusieurs composants catalytiquement actifs de Sn, Cu et Ni, et on met en contact avec un composé soluble de Ru et un composé soluble de Co, dans une étape b), simultanément ou successivement, le précurseur de catalyseur fabriqué dans l'étape a), le précurseur de catalyseur qui est fabriqué dans l'étape a) contenant en outre des composants catalytiquement actifs de Co.

2. Procédé selon la revendication 1, **caractérisé en ce que** le précurseur de catalyseur est, dans l'étape a), fabriqué par précipitation mixte et, avant la mise en contact avec Ru et Co dans l'étape b), contient une quantité de 1 à 95 % en poids de composants catalytiquement actifs de Sn, Cu et/ou Ni, calculés respectivement en CuO, NiO et SnO, et chacun par rapport à la masse totale du précurseur de catalyseur.

3. Procédé selon la revendication 1, **caractérisé en ce que** le précurseur de catalyseur est, dans l'étape a), fabriqué par précipitation et, avant la mise en contact avec Ru et Co dans l'étape b), contient une quantité de 5 à 95 % en poids d'un matériau support et une quantité de 5 à 90 % en poids de composants catalytiquement actifs Sn, Cu et/ou Ni, calculés respectivement en CuO, NiO ou SnO, et chacun par rapport à la masse totale du précurseur de catalyseur.

4. Procédé selon la revendication 1, **caractérisé en ce que** le précurseur de catalyseur est fabriqué dans l'étape a) par imprégnation et, avant la mise en contact avec Ru et Co dans l'étape b), contient une quantité de 50 à 99 % en poids du matériau support et une quantité de 1 à 50 % en poids de composants catalytiquement actifs de Sn, Cu et/ou Ni, calculés respectivement en CuO, NiO et SnO, et chacun par rapport à la masse totale du précurseur de catalyseur.

5. Procédé selon la revendication 1, **caractérisé en ce que** le précurseur de catalyseur fabriqué dans l'étape a) contient
- 10 à 75 % en poids de composants catalytiquement actifs de zirconium, calculés en ZrO₂ ;
- 1 à 30 % en poids de composants catalytiquement actifs de cuivre, calculés en CuO,
- 10 à 70 % en poids de composants catalytiquement actifs de nickel, calculés en NiO,
- 0,1 à 10 % en poids de composants catalytiquement actifs d'un ou plusieurs métaux choisis parmi Sb, Pb, Bi et In, calculés chacun respectivement en Sb₂O₃, PbO, Bi₂O₃ et In₂O₃,
par rapport à la masse totale du précurseur de catalyseur.

6. Procédé selon la revendication 1, **caractérisé en ce que** le précurseur de catalyseur fabriqué dans l'étape a) contient
- 10 à 75 % en poids de composants catalytiquement actifs de zirconium, calculés en ZrO₂,
- 1 à 30 % en poids de composants catalytiquement actifs de cuivre, calculés en CuO,
- 10 à 70 % en poids de composants catalytiquement actifs de nickel, calculés en NiO,
- 10 à 50 % en poids de composants catalytiquement actifs de cobalt, calculés en CoO, et
- 0,1 à 10 % en poids de composants catalytiquement actifs d'un ou plusieurs métaux choisis parmi Pb, Bi, Sn, Sb et In, chacun calculés respectivement en PbO, Bi₂O₃, SnO, Sb₂O₃ et In₂O₃,
par rapport à la masse totale du précurseur de catalyseur.

7. Procédé selon la revendication 1, **caractérisé en ce que** le précurseur de catalyseur fabriqué dans l'étape a) contient
- 20 à 70 % en poids de composants catalytiquement actifs de zirconium, calculés en ZrO₂,
- 15 à 60 % en poids de composants catalytiquement actifs de nickel, calculés en NiO,
- 0,5 à 14 % en poids de composants catalytiquement actifs de fer, calculés en Fe₂O₃, et
- 0,2 à 5,5 % en poids de composants catalytiquement actifs d'étain, de plomb, de bismuth, de molybdène, d'antimoine et/ou de phosphore, chacun calculés respectivement en SnO, PbO, Bi₂O₃, MoO₃, Sb₂O₃ et H₃PO₄,
par rapport à la masse totale du précurseur de catalyseur.

8. Procédé selon la revendication 1, **caractérisé en ce que** le précurseur de catalyseur fabriqué dans l'étape a) contient
- 20 à 85 % en poids de composants catalytiquement actifs de zirconium, calculés en ZrO₂,
- 0,2 à 25 % en poids de composants catalytiquement actifs de cuivre, calculés en CuO,
- 0,2 à 45 % en poids de composants catalytiquement actifs de nickel, calculés en NiO,
- 0,2 à 40 % en poids de composants catalytiquement actifs de cobalt, calculés en CoO,
- 0,1 à 5 % en poids de composants catalytiquement actifs de fer, calculés en Fe₂O₃, et
- 0,1 à 5,0 % en poids de composants catalytiquement actifs de plomb, d'étain, de bismuth et/ou d'antimoine, chacun calculés respectivement en PbO, SnO, Bi₂O₃ et Sb₂O₃,
par rapport à la masse totale du précurseur de catalyseur.

9. Procédé selon la revendication 1, **caractérisé en ce que** le précurseur de catalyseur fabriqué dans l'étape a) contient
- 46 à 65 % en poids de composants catalytiquement actifs de zirconium, calculés en ZrO₂,
- 5,5 à 18 % en poids de composants catalytiquement actifs de cuivre, calculés en CuO,
- 20 à 45 % en poids de composants catalytiquement actifs de nickel, calculés en NiO,
- 1,0 à 5,0 % en poids de composants catalytiquement actifs de cobalt, calculés en CoO, et
- 0,2 à 5,0 % en poids de composants catalytiquement actifs de vanadium, de niobium, de soufre, de phosphore, de galium, de bore, de tungstène, de plomb et/ou d'antimoine, chacun calculés respectivement en V₂O₅, Nb₂O₅, H₂SO₄, H₃PO₄, Ga₂O₃, B₂O₃, WO₃, PbO et Sb₂O₃,
par rapport à la masse totale du précurseur de catalyseur.

10. Procédé selon la revendication 1, **caractérisé en ce que** le précurseur de catalyseur fabriqué dans l'étape a) contient
- 0,2 à 5,0 % en poids de composants catalytiquement actifs d'étain, calculés en SnO,
- 10 à 30 % en poids de composants catalytiquement actifs de cobalt, calculés en CoO,
- 15 à 80 % en poids de composants catalytiquement actifs d'aluminium, calculés en Al₂O₃,
- 1 à 20 % en poids de composants catalytiquement actifs de cuivre, calculés en CuO,
- 5 à 35 % en poids de composants catalytiquement actifs de nickel, calculés en NiO, et
- 0,2 à 5,0 % en poids de composants catalytiquement actifs d'yttrium, de lanthane, de cérium et/ou d'hafnium, chacun calculés respectivement en Y₂O₃, La₂O₃, Ce₂O₃ et Hf₂O₃,
par rapport à la masse totale du précurseur de catalyseur.

11. Procédé selon la revendication 1, **caractérisé en ce que** le précurseur de catalyseur fabriqué dans l'étape a) contient
- 0,2 à 5 % en poids de composants catalytiquement actifs d'étain, calculés en SnO,
- 15 à 80 % en poids de composants catalytiquement actifs d'aluminium, calculés en Al₂O₃,
- 1 à 20 % en poids de composants catalytiquement actifs de cuivre, calculés en CuO
- 5 à 35 % en poids de composants catalytiquement actifs de nickel, calculés en NiO, et
- 5 à 35 % en poids de composants catalytiquement actifs de cobalt, calculés en CoO,
par rapport à la masse totale du précurseur de catalyseur.

12. Procédé selon la revendication 11, **caractérisé en ce que** la fabrication du précurseur de catalyseur a lieu en présence de nitrate d'étain et d'un complexant.

13. Procédé selon au moins l'une des revendications 1 à 12, **caractérisé en ce que** le précurseur de catalyseur est, dans l'étape b), mis en contact en même temps que le composé soluble de Ru et que le composé soluble de Co.

14. Procédé selon au moins l'une des revendications 1 à 13, **caractérisé en ce que** la concentration du composé soluble de Ru, avec lequel le précurseur de catalyseur fabriqué dans l'étape a) est mis en contact dans l'étape b), est comprise dans la plage de 0,1 à 50 % en poids, et que la concentration du composé soluble de Co, avec lequel le précurseur de catalyseur est mis en contact dans l'étape b), est comprise dans la plage de 0,1 à 20 % en poids.

15. Procédé selon au moins l'une des revendications 1 à 14, **caractérisé en ce que** la réaction de l'éthylèneglycol et/ou de la monoéthanolamine avec l'ammoniac en phase liquide a lieu sous une pression de 5 à 30 MPa et à une température dans la plage de 80 à 350 °C.
